# EUROPÄISCHE PATENTSCHRIFT

(11) **EP 0 950 750 B1**
(45) Veröffentlichungstag und Bekanntmachung des Hinweises auf die Patenterteilung: **22.10.2003**
(21) Anmeldenummer: 99810284.2
(22) Anmeldetag: 07.04.1999
(51) Int. Cl.: D06M 13/358, C07D 251/50, C07D 251/70

(54) **Verfahren zur Behandlung von Cellulosefasern**
Process for the treatment of cellulose fibers
Procédé de traitement de fibres de cellulose

(30) Priorität: 14.04.1998 EP 98810315; 19.05.1998 CH 109698
(43) Veröffentlichungstag der Anmeldung: 20.10.1999
(73) Patentinhaber: LENZING AKTIENGESELLSCHAFT, 4860 Lenzing (AT)
(72) Erfinder: Aeschlimann, Peter, 4123 Allschwil (CH); Müller, Bernhard, 79588 Efringen-Kirchen (DE)
(74) Vertreter: Schwarz, Albin, Dr.

(56) Entgegenhaltungen:
- EP-A- 0 538 977
- EP-A- 0 704 444
- EP-A- 0 882 836
- WO-A-94/09191
- CH-A- 385 780
- DE-A- 19 611 668
- DE-A- 19 720 683

## Beschreibung

Die vorliegende Erfindung betrifft ein Verfahren zur Verringerung der Fibrillierungsneigung bei Lyocell-Cellulosefasern.

Als Lyocell werden solche Faser bezeichnet, die durch ein Verfahren erhalten werden, bei dem die Cellulose in einem organischen Lösungsmittel, einer Kombination eines organischen Lösungsmittels mit einem anorganischen Salz oder in wässrigen Salzlösungen gelöst, und anschliessend aus dieser Lösung gesponnen wird.

Die Brauchbarkeit von aus den genannten Fasern hergestellten Flächengebilden, z.B. Textilmaterialien, wird jedoch durch die ausgeprägte Neigung dieser Fasern, im nassen Zustand zu fibrillieren, stark eingeschränkt. Unter Fibrillierung wird das Aufbrechen der nassen Faser in Längsrichtung bei z.B. mechanischer Beanspruchung bis zum Ablösen von Fibrillen entlang der Faseroberfläche verstanden, wodurch die Faser ein haariges oder pelziges Aussehens erhält. Ein aus diesen Fasern hergestelltes Gewebe verliert ausserdem nach einigen Wäschen stark an Farbintensität.

Es besteht somit ein Bedarf nach einem Verfahren, das die Fibrillierung bei Lyocell-Fasem verringert oder diese vollständig unterdrückt.

Es hat sich überaschenderweise gezeigt, dass durch das erfindungsgemässe Verfahren die Fibrillierungsneigung der behandelten Lyocell-Fasern stark verringert wird.

Gegenstand der vorliegenden Erfindung ist somit ein Verfahren zur Behandlung von Lyocell-Cellulosefasern, dadurch gekennzeichnet, dass man die Lyocell-Cellulosefaser mit mindestens einer Verbindung der Formel 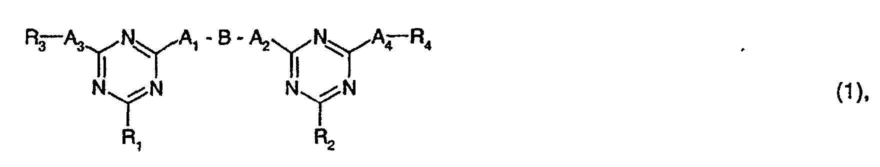 behandelt, worin
R₁ und R₂ unabhängig voneinander je Halogen oder ein durch Sulfo substituierter Phenylaminorest sind, wobei mindestens einer der beiden Substituenten R₁ und R₂ die Bedeutung von Halogen hat,
R₃ und R₄ unabhängig voneinander je unsubstituiertes oder durch C₁-C₄-Alkyl, C₁-C₄-Alkoxy, Acylamino, Sulfo, -SO₂-CH=CH₂, -SO₂-CH₂CH₂-OSO₃H, -NH-CO-CBr=CH₂ oder -NH-CO-CHBr-CH₂Br substituiertes Phenyl ist,
A₁ und A₂ unabhängig voneinander je -O-, -S- oder -NR₅- sind, worin R₅ Wasserstoff oder C₁-C₄-Alkyl bedeutet,
A₃ und A₄ unabhängig voneinander je -O-, -S- oder -NR₅- sind , oder -A₃-R₃ Halogen und/oder -A₄-R₄ Halogen bedeutet, worin R₅ Wasserstoff oder C₁-C₄-Alkyl bedeutet und
B ein aromatisches Brückenglied oder -A₁-B-A₂- ein Brückenglied der Formel -NH-CH₂-CH(CH₃)-NH- ist, mit der Massgabe, dass die Verbindung der Formel (1) mindestens einen Dihalogentriazinrest, mindestens zwei Monohalogentriazinreste oder mindestens einen, vorzugsweise mindestens zwei, voneinander unabhängige Substituenten aus der Gruppe, enthaltend -SO₂-CH=CH₂, -SO₂-CH₂CH₂-OSO₃H, -NH-CO-CBr=CH₂ und -NH-CO-CHBr-CH₂Br, enthalten muss.

C₁-C₄-Alkyl als Phenylsubstituent in R₃, und R₄ ist Methyl, Ethyl, n-Propyl, iso-Propyl, n-Butyl, sec.-Butyl, iso-Butyl oder tert.-Butyl.
R₅ als C₁-C₄-Alkyl ist Methyl, Ethyl, Propyl, iso-Propyl, n-Butyl, sec.-Butyl, iso-Butyl oder tert.-Butyl.
C₁-C₄-Alkoxy als Phenylsubstituent in R₃ und R₄ ist z.B. Methoxy, Ethoxy, Propoxy oder Butoxy.
Acylamino als Phenylsubstituent in R₃ und R₄ ist z.B. Acetylamino.
A₃-R₃ und A₄-R₄ als Halogen bedeutet bevorzugt Fluor und insbesondere Chlor.

B als aromatisches Brückenglied ist z.B. ein Rest der Formel 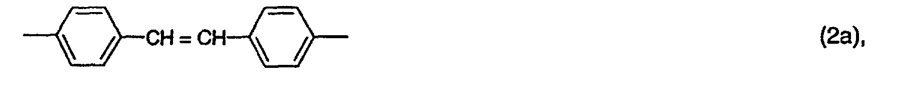 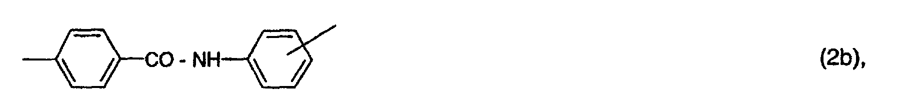 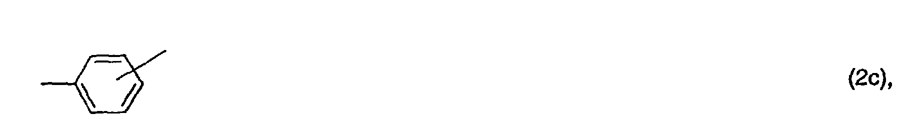 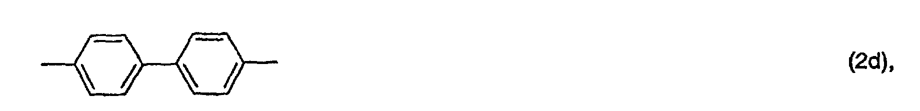 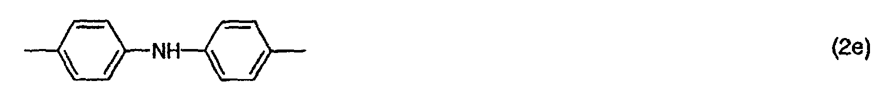 oder 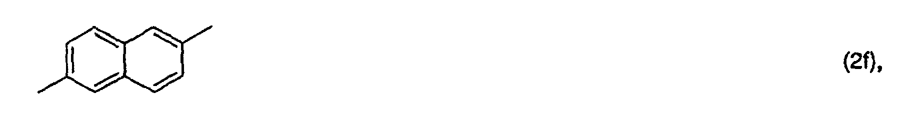 wobei die Phenylreste in den Formeln (2a) bis (2f) ein- oder mehrfach durch Sulfo substituiert sein können.

Halogen als R₁ und R₂ bedeutet unabhängig voneinander Fluor oder Chlor.

Als R₁ ist Halogen, insbesondere Chlor bevorzugt.
Als R₂ ist Halogen, insbesondere Chlor bevorzugt.
Als R₃ ist ein durch C₁-C₄-Alkyl, C₁-C₄-Alkoxy, Acylamino, Sulfo, -SO₂-CH=CH₂, -SO₂-CH₂CH₂-OSO₃H, -NH-CO-CBr=CH₂ oder -NH-CO-CHBr-CH₂Br substituiertes Phenyl bevorzugt, wobei der Phenylrest ein- oder mehrfach durch die genannten Substituenten substituiert werden kann.
Als R₄ ist ein durch C₁-C₄-Alkyl, C₁-C₄-Alkoxy, Acylamino, Sulfo, -SO₂-CH=CH₂, -SO₂-CH₂CH₂-OSO₃H, -NH-CO-CBr=CH₂ oder -NH-CO-CHBr-CH₂Br substituiertes Phenyl bevorzugt, wobei der Phenylrest ein- oder mehrfach durch die genannten Substituenten substituiert werden kann.
Als A₁ ist -NR₅- bevorzugt.
Als A₂ ist -NR₅- bevorzugt.
Als A₃ ist -NR₅- bevorzugt.
Als A₄ ist -NR₅- bevorzugt.
Als R₅ ist Wasserstoff bevorzugt.

Wichtig für das erfindungsgemässe Verfahren sind die Verbindungen der Formel (1), worin A₁, A₂, A₃ und A₄ -NH- bedeuten,
B ein Rest der Formel

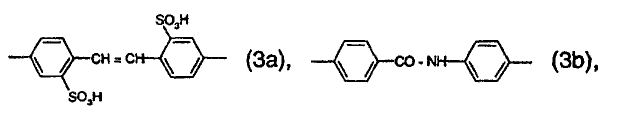

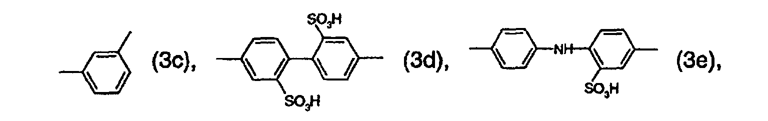

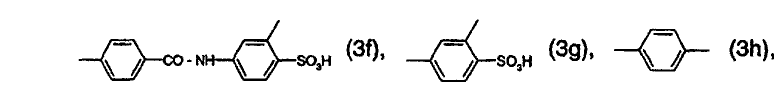

-CH₂-CH(CH₃)- (3i)

oder 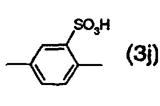 ist, R₁ Chlor, Fluor oder 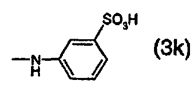 ist, R₂ Chlor oder Fluor ist, R₃ ein Rest der Formel 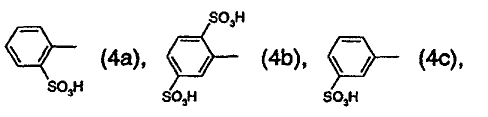 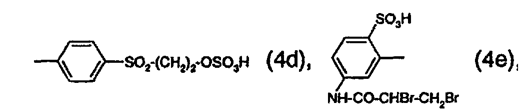 oder 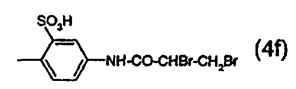 ist, und R₄ ein Rest der Formel (4a), (4b), (4d), (4e), (4f), , 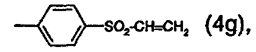 oder 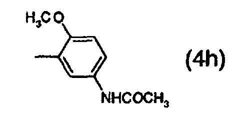 ist, oder A₃-R₃ und/oder A₄-R₄ Chlor bedeutet.

Besonders wichtig für das erfindungsgemässe Verfahren sind die Verbindungen der Formeln 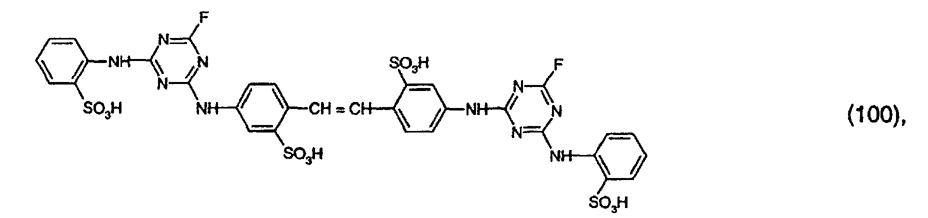 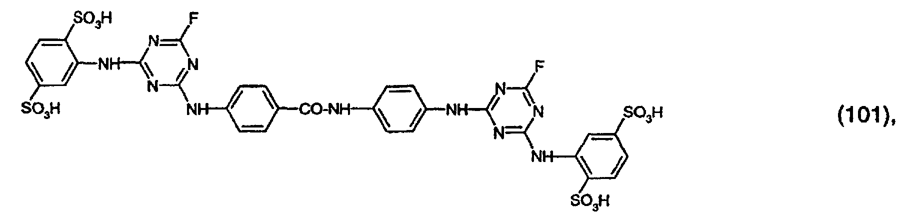 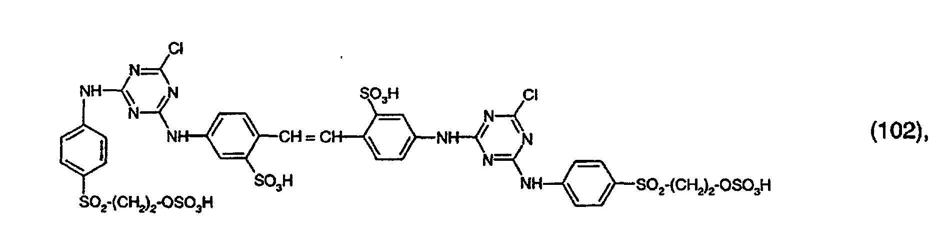 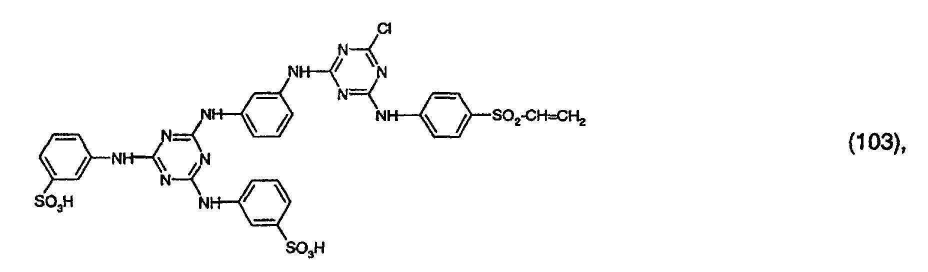 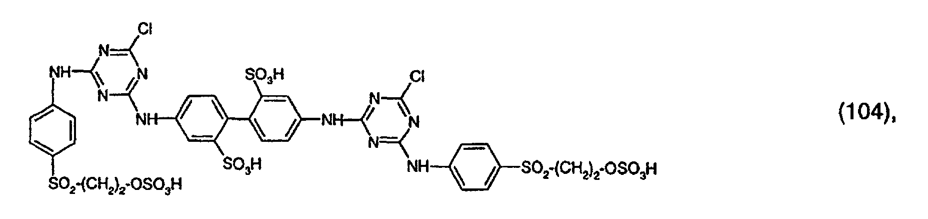 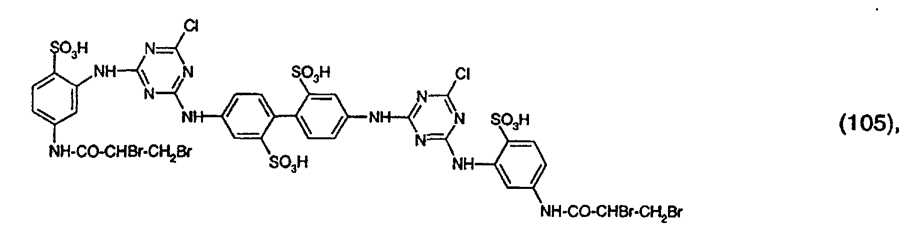 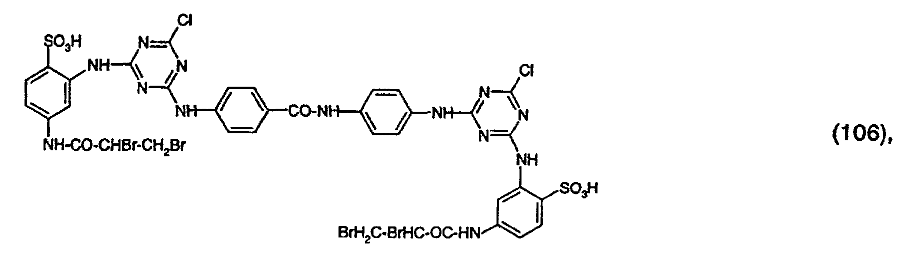 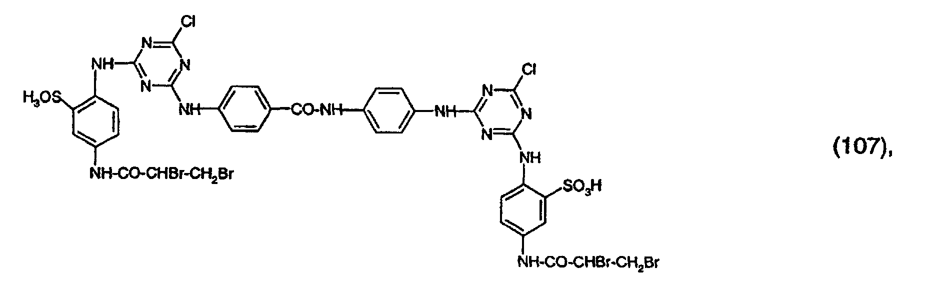 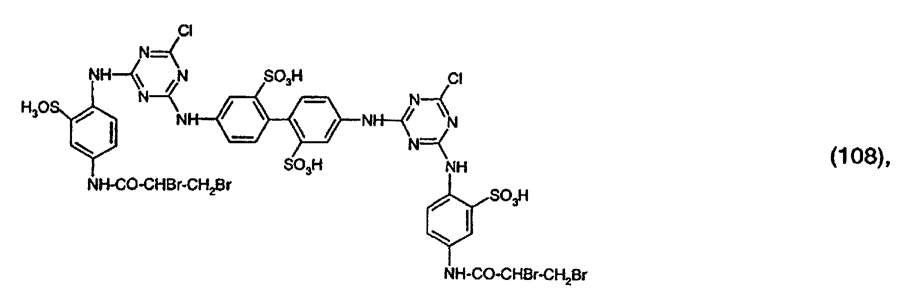 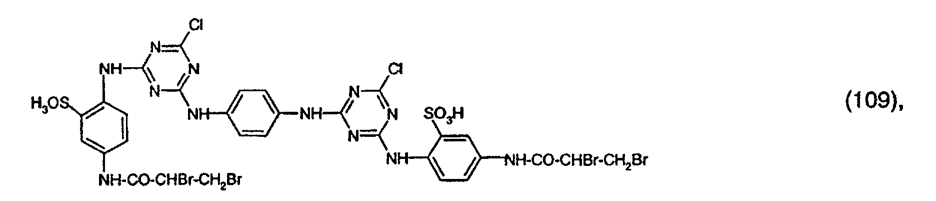 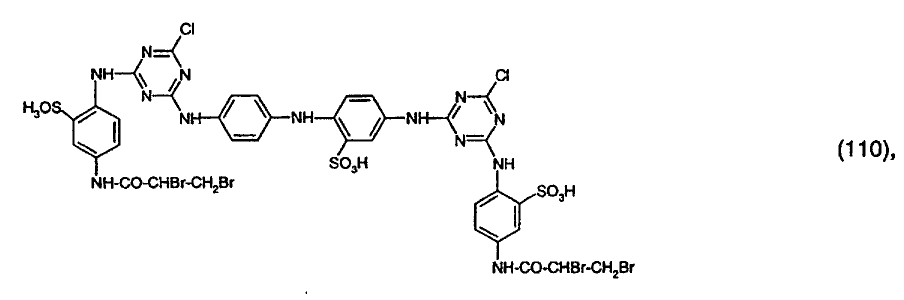 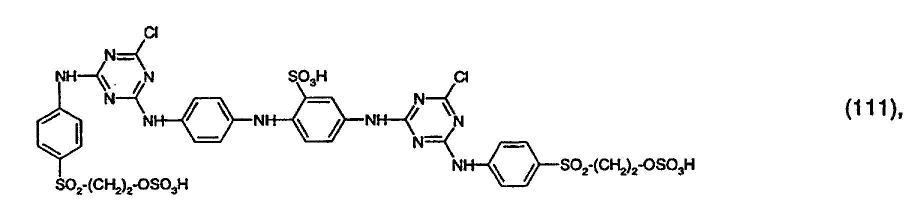 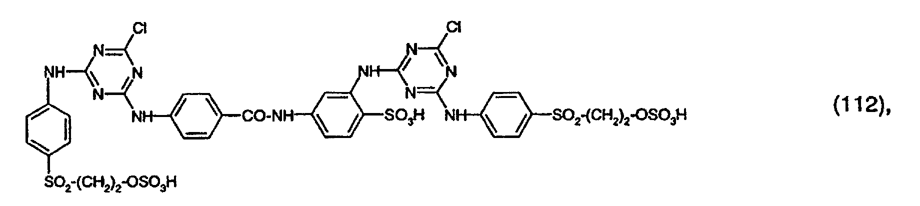 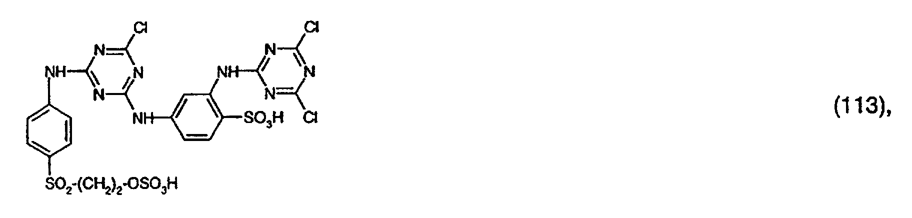 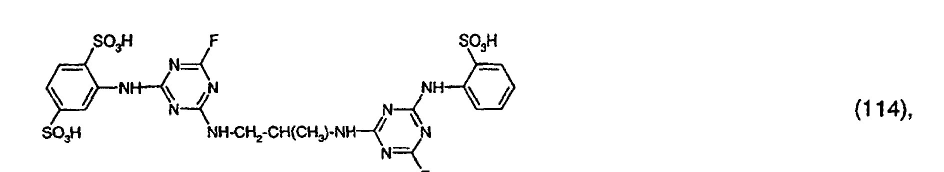 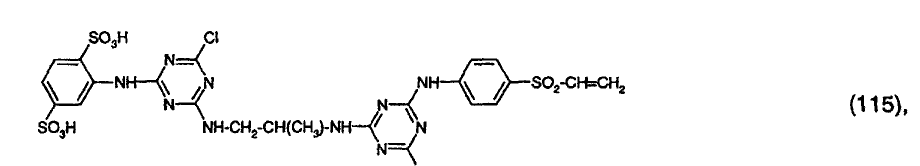 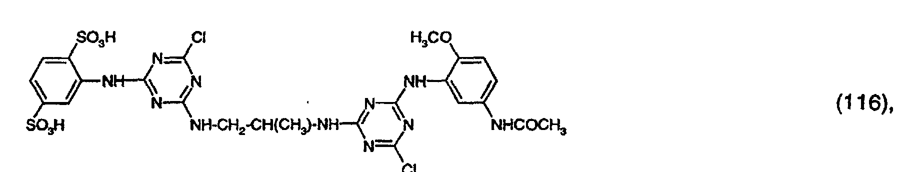 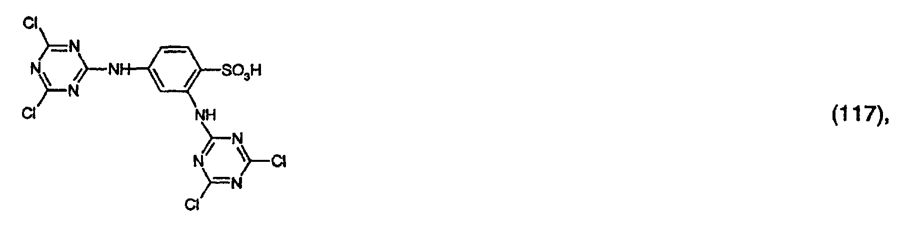 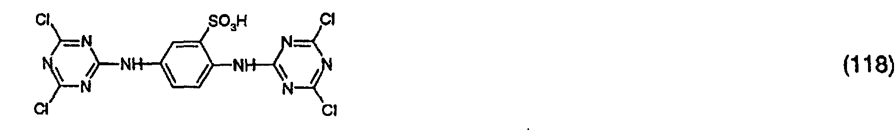 und

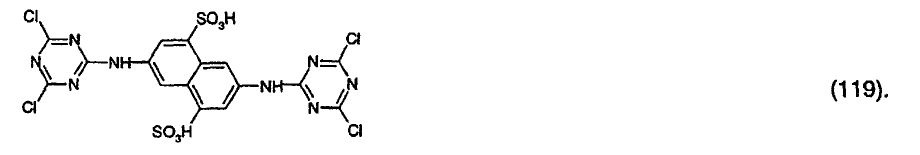

Ganz besonders wichtig für das erfindungsgemässe Verfahren sind die Verbindungen der Formeln (100), (101), (102), (103), (105), (108), (114), (117), (118) und (119).

Die Verbindungen der Formeln (100), (101), (103), (105) bis (116) und (119) sind neu und stellen einen weiteren Gegenstand der vorliegenden Erfindung dar.

Die Herstellung der in dem erfindungsgemässen Verfahren verwendeten Verbindungen der Formel (1), geschieht nach an sich bekannten Methoden, z. B., indem man ein Trichlortriazin der Formel 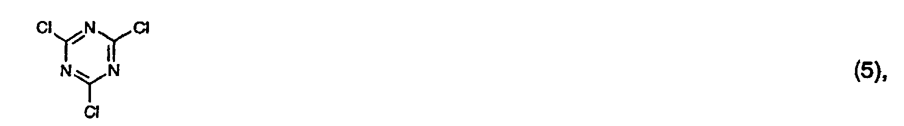 oder ein Trifluorotriazin der Formel 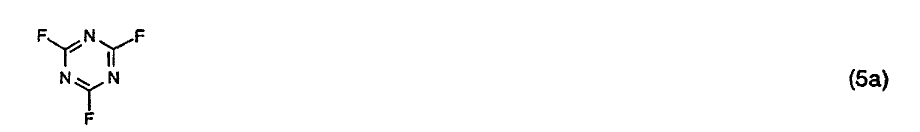 mit äquimolarer Menge einer Verbindung der Formel

R₃-A₃H (6),

worin R₃ und A₃ die unter der Formel (1) angegebene Bedeutung haben, und anschliessend mit einer Verbindung der Formel

HA₁-B-A₂H (7),

worin A₁, A₂ und B die unter der Formel (1) angegebene Bedeutung haben,
zu einem Zwischenprodukt der Formel 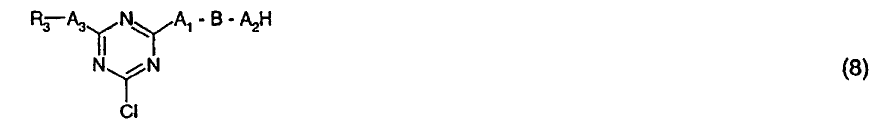 umsetzt.

Danach setzt man ein zweites Trichlortriazin der Formel (5) oder ein zweites Trifluorotriazin der Formel (5a) gegebenenfalls mit äquimolarer Menge einer Verbindung der Formel

R₄-A₄H (9),

worin A₄ und R4 die unter der Formel (1) angegebene Bedeutung haben,
und anschliessend mit dem Zwischenprodukt der Formel (8) um, und isoliert die entstehende Verbindung der Formel 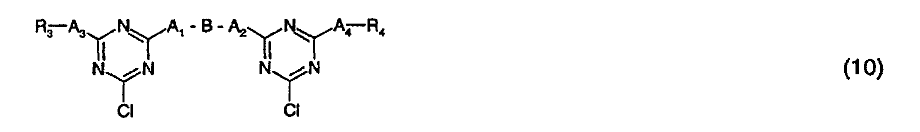 oder der Formel

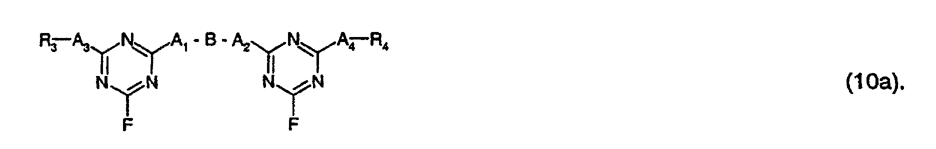

Oder man setzt z.B. ein Trichlortriazin der Formel (5) oder ein Trifluorotriazin der Formel (5a) mit einer Verbindung der Formel (7) im Mol-Verhältniss 2:1 um.

Die Herstellung der neuen Verbindungen der Formeln (100),), (101), (103), (105) bis (116) und (119) geschieht in analoger Weise, indem man ein Trichlortriazin der Formel (5) mit einer Verbindung der Formel 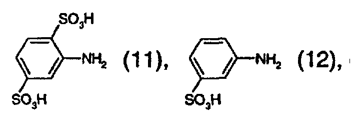 oder 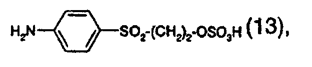 und anschliessend mit einer Verbindung der Formel 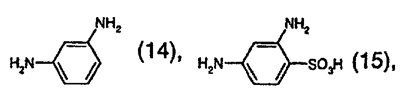 oder

H₂N-CH₂-CH(CH₃)-NH₂ (16),

umsetzt, und mit einem zweiten Trichlortriazin der Formel (5), welches gegebenenfalls vorgängig mit einer Verbindung der Formel 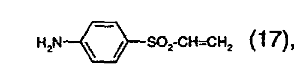 oder 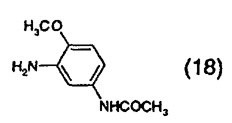 umgesetzt wurde, kondensiert, oder, falls A₃-R₃ = A₄-R₄, man Trichlortriazin der Formel (5) mit einer Verbindung der Formel (13), 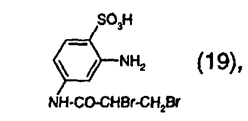 oder 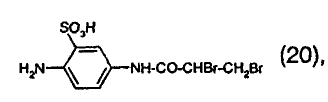 umsetzt und das entstehende Zwischenprodukt im Mol-Verhältnis 2:1 mit einer Verbindung der Formel 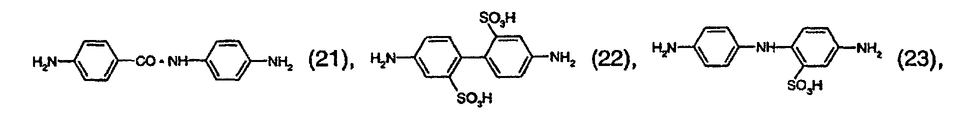 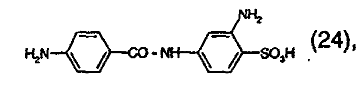 oder 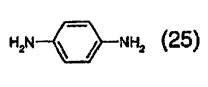 kondensiert, oder, falls A₃-R₃ = A₄-R₄ = Halogen, man Trichlortriazin der Formel (5) im Mol-Verhältnis 2:1 mit einer Verbindung der Formel (15) oder 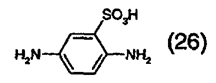 kondensiert, oder indem man ein Trifluorotriazin der Formel (5a) mit einer Verbindung der Formel 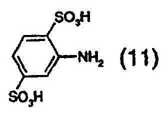 und anschliessend mit einer Verbindung der Formel (16), umsetzt, und mit einem zweiten Trifluorotriazin der Formel (5a), welches vorgängig mit einer Verbindung der Formel 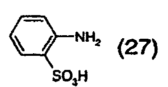 umgesetzt wurde, kondensiert, oder, falls A₃-R₃ = A₄-R₄, man Trifluorotriazin der Formel (5a) mit einer Verbindung der Formel (27) oder (11) umsetzt und das entstehende Zwischenprodukt im Mol-Verhältnis 2:1 mit einer Verbindung der Formel 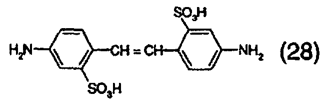 oder (21), kondensiert.

Verbindungen der Formeln (11) bis (28) sind bekannt und können nach an sich bekannten Methoden hergestellt werden.

Eine zweckmässige Ausführungsform des erfindungsgemässen Verfahrens ist dadurch gekennzeichnet, dass die Lyocell-Cellulosefasem mit den erfindungsgemäss verwendeten Verbindungen in einem alkalischen Milieu behandelt werden.
Das alkalische Milieu wird vorzugsweise von einem Alkylicarbonat und/oder einem Alkalihydroxid, wie z.B. Natriumcarbonat, Natriumhydroxid oder Kaliumhydroxid, gebildet.

Die Behandlung kann dabei sowohl an ungefärbten Fasern, als auch an zu färbenden Fasern vor, während oder unmittelbar nach einem Färbeprozess, durchgeführt werden.
Werden die erfindungsgemäss verwendeten Verbindungen an ungefärbten Fasern appliziert, so kann es entweder mittels eines Behandlungsbades oder direkt nach dem Spinnvorgang an den frisch gesponnenen, noch nicht getrockneten Fasern (sog. "never dried"-Faser, wie sie z.B. in der EP-A-0 538 977 auf der Seite 4, oder in der US-A-5,580,354 in der Spalte 4 beschrieben sind) geschehen.
Werden die erfindungsgemäss verwendeten Verbindungen im Laufe eines Färbeprozesses eingesetzt, so geschieht dies vorteilhafterweise vor oder während des eigentlichen Färbevorganges.
Die erfindungsgemäss verwendeten Verbindungen können dabei in einem separaten Behandlungsbad, oder vorzugsweise zusammen mit den verwendeten Farbstoffen in einem Färbebad bei den jeweiligen Färbebedingungen auf die Faser appliziert werden.
Werden die erfindungsgemäss verwendeten Verbindungen aus einem separaten Behandlungsbad auf die Faser gebracht, so kann dies bei einer Temperatur von 15 bis 140° C, vorzugsweise von 40 bis 100° C während 10 bis 120 Minuten, vorzugsweise 30 bis 90 Minuten, erfolgen. Eine weitere vorteilhafte Ausführung des erfindungsgemässen Verfahrens besteht darin, die zu behandelnden Faser mit einer wässrigen Lösung der erfindungsgemäss verwendeten Verbindungen zu benetzen und anschliessend während 5 bis 60, vorzugsweise 10 bis 30 Sekunden einer Dampfbehandlung bei 90 bis 130° C zu unterziehen. Die Benetzung der Faser kann sowohl durch Eintauchen in ein Bad als auch durch Besprühen der Faser stattfinden.

Die Lyocell-Cellulosefasem können als solche, als Garn, oder in Form von Flächengebilden, wie z.B. Gewebe, Gestricke oder Gewirke vorliegen und behandelt werden.
Die Mengen in denen die erfindungsgemäss verwendeten Verbindungen in den Behandlungs- oder Färbeflotten eingesetzt werden, liegen normalerweise zwischen 0,1 und 15 Gew.-%, insbesondere zwischen 1 und 10 Gew.-%, vor allem zwischen 2 und 6 Gew.-% bezogen auf das Gewicht der Faser.
Einen weiteren Gegenstand der vorliegenden Erfindung stellt die Verwendung der Verbindungen der Formel (1) zur Verringerung der Fibrillierung bei der Lyocell-Cellulose dar.
Die Fibrillierungneigung der unbehandelten und behandelten Fasern wird nach einem modifiziertem Martindale Scheuertest, bei dem das genässte Gewebemuster bis zur ersten Lochbildung unter definierter Belastung gescheuert wird, beurteilt. Die Anzahl der Scheuertouren bis zur Lochbildung ergibt die Nassscheuerbeständigkeit, die als Massstab für die Fibrillierung dient.
Die nachfolgende Beispiele dienen der Erläuterung der Erfindung. Die Temperaturen sind in Celsiusgraden angegeben, Teile sind Gewichtsteile, und Prozentangaben beziehen sich auf Gew.-%, sofern nicht anders vermerkt. Gewichtsteile stehen zu Volumenteilen im Verhältnis von Kilogramm zu Liter.

### Beispiel 1:

14,35 g Anilin-2,5 disulfosäure werden in einem Laborreaktionsgefäss in 100 Teilen Wasser neutral gelöst, mit 1 g Natriumdihydrogenphosphat versetzt und auf 0°C gekühlt. Unter intensivem Rühren tropft man innerhalb von 10 Minuten 4,5 ml Cyanurfluorid dazu und hält die Temperatur durch Kühlung bei 0°C. Der pH-Wert wird durch Zudosierung von 5-normaler wässriger Natriumhydroxidlösung bei 6,0 gehalten. Nach Beendigung der Kondensation setzt man eine Lösung von 3,7 g 1,2-Diaminopropan in 25 ml Wasser und 5 ml einer 10-normalen Salzsäure zu und lässt bei 2 bis 5°C und pH 6,0 während 5 Stunden kondensieren, wobei der pH-Wert mit einer 5-normalen wässriger Natriumhydroxidlösung bei 6,0 gehalten wird. Es resultiert eine Verbindung der Formel 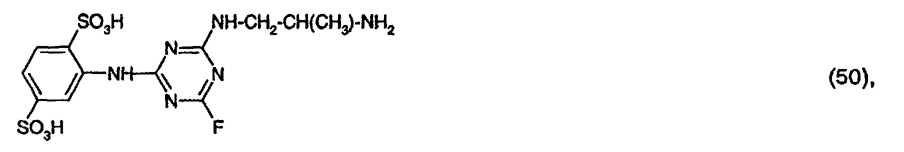 welche als Zwischenprodukt in guter Ausbeute und Reinheit als eine Lösung vorliegt.
In einem separaten Becherglas werden 8,6 g Anilin 2-Sulfosäure in 100 Teilen Wasser angerührt und mit 10 ml einer 5 normalen wässriger Natriumhydroxidlösung neutral gelöst. Nun tropft man innert 10 Minuten unter sehr intensivem Rühren bei 0°C und pH 6 haltend 4,5 ml Cyanurfluorid zu. Die Kondensation ist nach 10 Minuten Rührdauer beendet.

Die erhaltene Lösung wird zu der oben beschriebenen Lösung der Verbindung der Formel (50) zugegeben, der pH-Wert auf 9,0 eingestellt und konstant gehalten. Man lässt die Tem peratur innert 30 Minuten auf 15°C ansteigen. Man erhält eine Lösung der Verbindung der Formel

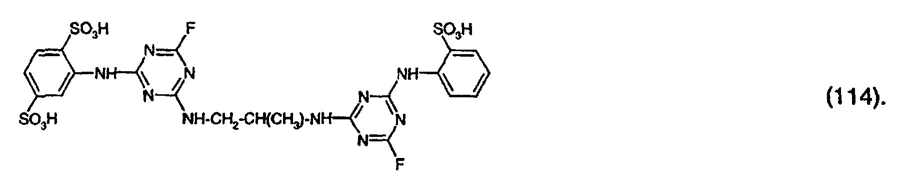

Zu dieser Lösung werden 1,8 g Natriumtripolyphosphat-Puffer zugesetzt und die Lösung wird bei pH 7,5 gefriergetrocknet.
Man erhält 42 g eines weisen, gut wasserlöslichen Pulvers der Verbindung der Formel (114).

### Beispiele 2 bis 5:

Verfährt man wie im Beispiel 1 angegeben, verwendet aber anstatt Cyanurfluorid die äquimolare Menge Cyanurchlorid, anstatt Anilin-2,5 disulfosäure die äquimolare Menge einer der Verbindungen der Formeln (11) bis (13), anstatt 1,2-Diaminopropan gegebenenfalls die äquimolare Menge einer der Verbindungen der Formeln (15) oder (25), und anstatt Anilin 2-Sulfosäure die äquimolare Menge einer der Verbindungen der Formeln (17) oder (18), so erhält man die in der Tabelle 1 aufgeführten Verbindungen der Formeln (103), (113), (115) und (116).

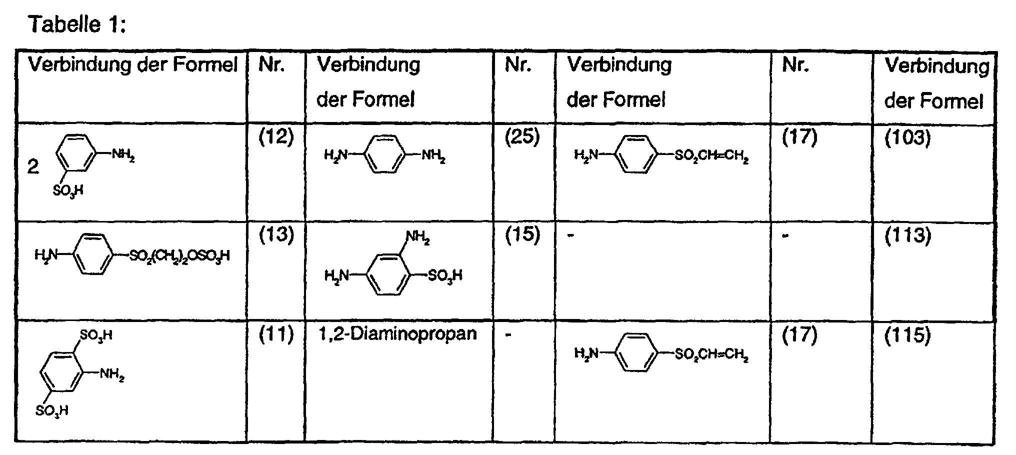

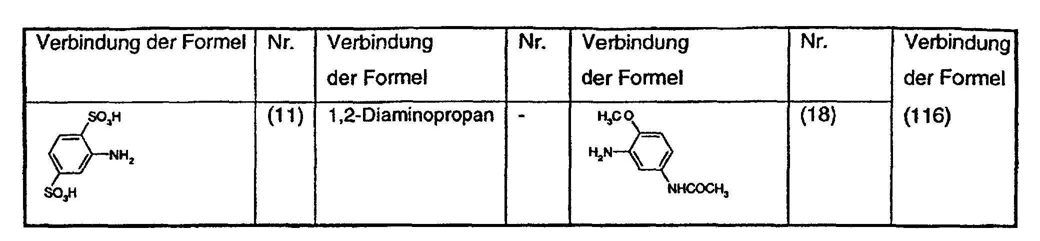

### Beispiel 6:

9,4 g Cyanurchlorid werden zusammen mit 100 g Eiswasser, 0.1 g eines handelsüblichen oberflächenwirksamen Hilfsmittels sowie 1,1g Natriumdihydrogenphosphat als Puffer in einem Laborreaktionsgefäss während einer Stunde bei 0°C intensiv verrührt.

In einem zweiten Laborreaktionsgefäss werden 14 g einer Verbindung der Formel 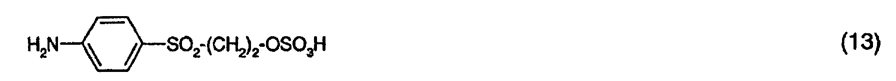 in 150 ml Wasser, welches 5,3 g Natriumcarbonat enthält, bei pH 6,0 und einer Temperatur von 30°C klar gelöst. Die entstandene Lösung wird auf ca 0°C gekühlt und anschliessend zu der Cyanurchloridsuspension zugegeben. Die Mischung wird danach bei pH 6,0 bei einer Temperatur zwischen 0 und 2°C solange gerührt, bis mittels einer HPLC-Probe keine Verbindung der Formel (13) nachgewiesen werden kann.
Nun trägt man zu der obigen Mischung 9,2 g einer Verbindung der Formel 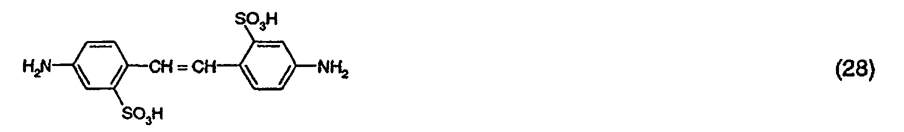 ein, und lässt die Temperatur langsam bis ca. 30 bis 35°C ansteigen, wobei der pH-Wert wiederum bei 6,0 gehalten wird.
Zur Vervollständigung der Kondensation rührt man bei 30 bis 35°C noch ca 30 Minuten nach. Die Reaktionslösung wird anschliessend durch Dialyse entsalzt und dann gefriergetrocknet. Man erhält 37 g eines hellen Pulvers der Verbindung der Formel

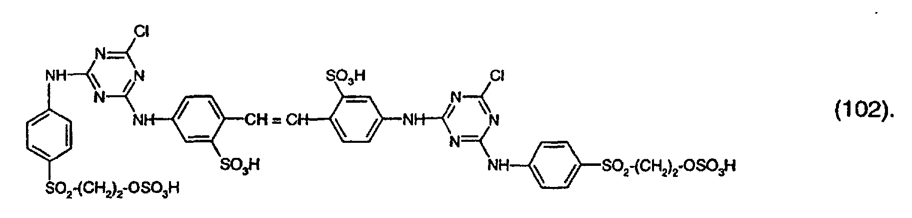

### Beispiel 7:

Verfährt man wie im Beispiel 6 angegeben, verwendet aber anstatt Cyanurchlorid die äquimolare Menge Cyanurfluorid, und anstatt der Verbindung der Formel (13) die äquimolare Menge der Verbindung der Formel (27) so erhält man die Verbindung der Formel (100).

### Beispiel 8:

Verfährt man wie im Beispiel 6 angegeben, verwendet aber anstatt Cyanurchlorid die äquimolare Menge Cyanurfluorid, und anstatt der Verbindung der Formel (13) die äquimolare Menge der Verbindung der Formel (11) und anstatt der Verbindung der Formel (28) die äquimolare Menge der Verbindung der Formel (21), so erhält man die Verbindung der Formel (101).

### Beispiele 9 bis 11:

Verfährt man wie im Beispiel 6 angegeben, verwendet aber anstatt der Verbindung der Formel (28) die äquimolare Menge einer der Verbindungen der Formeln (22), (23) und (24), so erhält man die Verbindungen der Formeln (104), (111) und (112).

### Beispiele 12 bis 17:

Verfährt man wie im Beispiel 6 angegeben, verwendet aber anstatt der Verbindung der Formel (28) die äquimolare Menge einer der Verbindungen der Formeln (21) (22), (23) und (25), und anstatt der Verbindung der Formel (13) die äquimolare Menge einer der Verbindungen der Formeln (19), oder (20) so erhält man die in der Tabelle 2 aufgeführten Verbindungen der Formeln (105) bis (110).

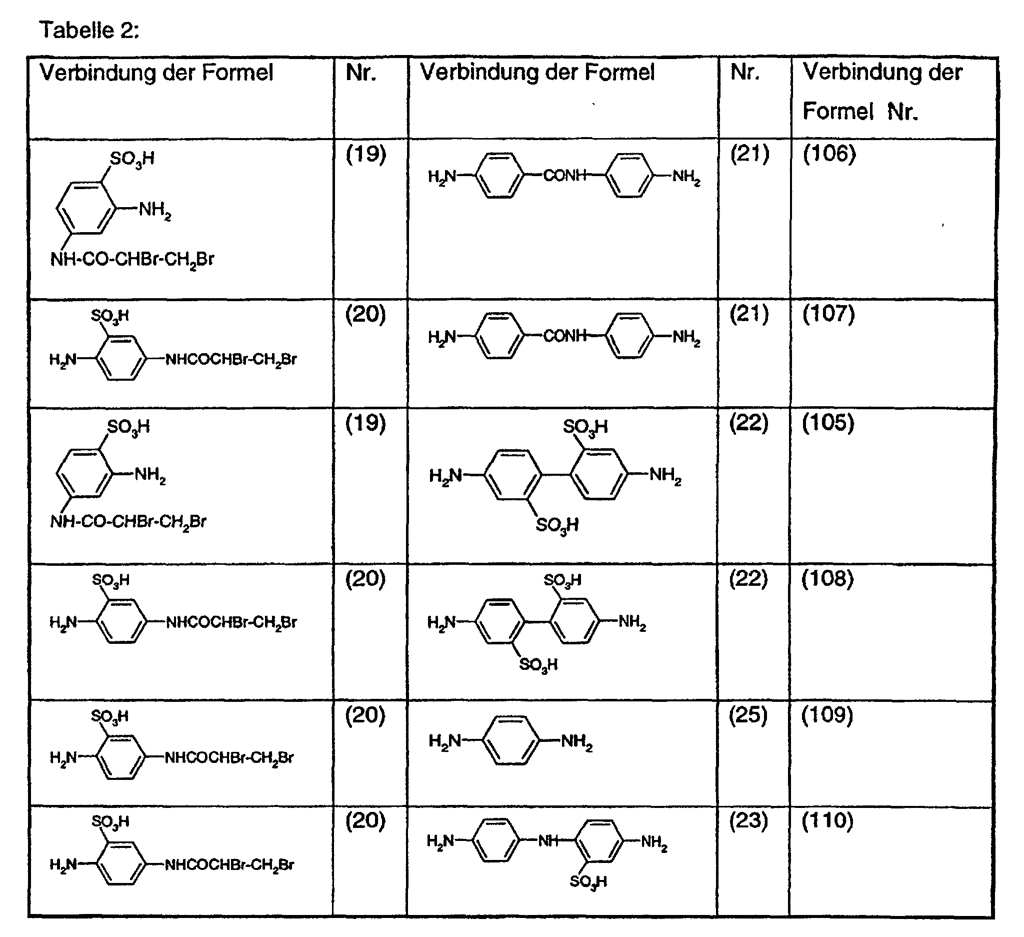

### Beispiel 18:

in einer Laborfärbeapparatur wird eine Flotte, bestehend aus
50 ml Wasser und
15 ml einer 20%-igen wässrigen Lösung von Natriumsulfat,
vorgelegt und auf 50° C aufgeheizt. Danach wird in die Flotte ein Stück von 10 Lyocell-Gewebe eingetaucht und die Temperatur mit einem Gradient von 2° C/Min auf 90° C erhöht. Während der Aufheizphase werden bei 70° C 0,6 g der Verbindung der Formel (114), gelöst in 27,5 ml Wasser, zugegeben. Das Lyocell-Gewebe wird bei 90° C 60 Minuten be-handelt. Anschliessend wird die Behandlungsflotte auf 70° C abgekühlt, versetzt mit 7,5 ml einer 20%-igen wässrigen Soda-Lösung und weitere 45 Minuten bei 70° C gehalten.

Danach wird das Bad abgelassen und das behandelte Lyocell-Gewebe mit Wasser gespült, in frischem, nur aus Wasser bestehendem, Bad 5 Minuten gekocht, nochmals kalt gespült und getrocknet. Das getrocknete Lyocell-Gewebe zeigt bei einem Martindale-Scheuertest eine ca. 1,5-fache Anzahl Scheuertouren im Vergleich zu unbehandeltem Lyocell-Gewebe.

Bei dieser Applikation kann das Lyocell-Gewebe auch gleichzeitig gefärbt werden, indem ein oder mehrere Reaktivfarbstoffe direkt nach Zugabe der Verbindung der Formel (114) zugegeben werden.

Verfährt man wie im Beispiel 18 beschrieben, verwendet aber anstatt 0,6 der Verbindung der Formel (114) die gleiche Menge einer der Verbindungen der Formeln (100) bis (113), und (115) bis (118), erhält man ebenfalls ein Lyocell-Gewebe mit hohen Martindale-Scheuertestwerten.

### Beispiel 19:

In einer Laborfärbeapparatur wird eine Flotte, bestehend aus
50 ml Wasser und
15 ml einer 20%-igen wässrigen Lösung von Natriumsulfat,
vorgelegt und auf 60° C aufgeheizt. Danach wird in die Flotte ein Stück von 10 Lyocell-Gewebe eingetaucht und 3 Minuten später 0,6 g der Verbindung der Formel (113) gelöst in 25,5 ml Wasser zugegeben. Nach weiteren 15 Minuten wird die Flotte mit 7,5 ml einer
20%-igen wässrigen Soda-Lösung versetzt. Danach hält man die Flotte 30 Minuten bei 60°C.
Dann versetzt man die Flotte mit 2 ml einer 3%-igen wässrigen NaOH-Lösung, hält weitere 10 Minuten bei 60° C und stellt anschliessend das Lyocell-Gewebe wie in Beispiel 18 beschrieben fertig.
Das fertiggestellte Lyocell-Gewebe kann anschliessend nach üblichen Verfahren, mit z.B. Reaktivfarbstoffen, gefärbt werden.
Das getrocknete Lyocell-Gewebe zeigt bei einem Martindale-Scheuertest eine ca. 1,5-fache Anzahl Scheuertouren im Vergleich zu unbehandeltem Lyocell-Gewebe.

Verfährt man wie im Beispiel 19 beschrieben, verwendet aber anstatt 0,6 g der Verbindung der Formel (113) die gleiche Menge einer der Verbindungen der Formeln (100) bis (112) und (114) bis (118), so erhält man ebenfalls ein Lyocell-Gewebe mit hohen Martindale-Scheuertestwerten.

### Beispiel 20:

Eine wässrige Flotte, enthaltend
42 g/l einer Verbindung der Formel 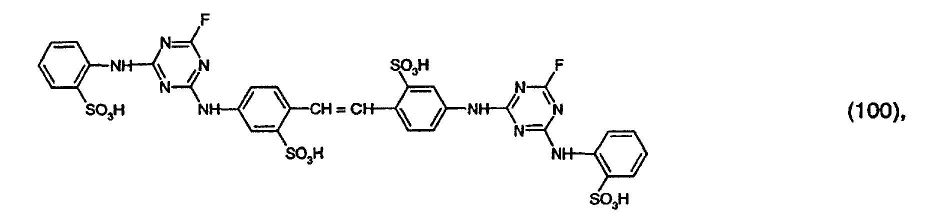 30 g/l kalziniertes Natriumcarbonat und
1 g/l eines handelsüblichen Netzmittels,
wird auf ein Lyocell-Gewebe auffoulardiert (Flottenaufnahme 70%). Das so behandelte Lyocell-Gewebe wird ohne Trocknung direkt während 8 Minuten in Sattdampf von 102° C weiterbehandelt. Danach wird das Lyocell-Gewebe mit Wasser gespült, in frischem, rein wässrigem Bad 5 Minuten gekocht, kalt gespült und getrocknet.
Das behandelte Lyocell-Gewebe zeigt bei einem Martindale-Scheuertest im Vergleich zu unbehandeltem Lyocell-Gewebe eine ca. 2-fache Anzahl Scheuertouren.

Verfährt man wie im Beispiel 19 beschrieben, verwendet aber anstatt 42 g der Verbindung der Formel (100) die gleiche Menge einer der Verbindungen der Formeln (101) bis (119), so erhält man ebenfalls ein Lyocell-Gewebe mit hohen Martindale-Scheuertestwerten.

## Patentansprüche

1. Verfahren zur Behandlung von Lyocell-Cellulosefasem, **dadurch gekennzeichnet, dass** man die Lyocell-Cellulosefaser mit mindestens einer Verbindung der Formel 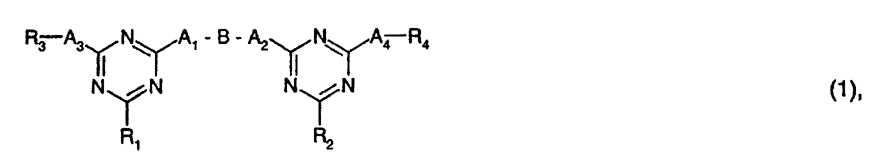 behandelt, worin
R₁ und R₂ unabhängig voneinander je Halogen oder ein durch Sulfo substituierter Phenylaminorest sind, wobei mindestens einer der beiden Substituenten R₁ und R₂ die Bedeutung von Halogen hat,
R₃ und R₄ unabhängig voneinander je unsubstituiertes oder durch C₁-C₄-Alkyl, C₁-C₄-Alkoxy, Acylamino, Sulfo, -SO₂-CH=CH₂, -SO₂-CH₂CH₂-OSO₃H, -NH-CO-CBr=CH₂ oder -NH-CO-CHBr-CH₂Br substituiertes Phenyl ist,
A₁ und A₂ unabhängig voneinander je -O-, -S- oder -NR₅- sind, worin R₅ Wasserstoff oder C₁-C₄-Alkyl bedeutet,
A₃ und A₄ unabhängig voneinander je -O-, -S- oder -NR₅- sind , oder -A₃-R₃ Halogen und/oder -A₄-R₄ Halogen bedeutet, worin R₅ Wasserstoff oder C₁-C₄-Alkyl bedeutet und
B ein aromatisches Brückenglied oder -A₁-B-A₂- ein Brückenglied der Formel -NH-CH₂-CH(CH₃)-NH- ist, mit der Massgabe, dass die Verbindung der Formel (1) mindestens einen Dihalogentriazinrest, mindestens zwei Monohalogentriazinreste oder mindestens einen, vorzugsweise mindestens zwei, voneinander unabhängige Substituenten aus der Gruppe, enthaltend -SO₂-CH=CH₂, -SO₂-CH₂CH₂-OSO₃H, -NH-CO-CBr=CH₂ und -NH-CO-CHBr-CH₂Br, enthalten muss.

2. Verfahren gemäss Anspruch 1, **dadurch gekennzeichnet, dass** die Verbindung der Formel (1) mindestens zwei voneinander unabhängige Substituenten aus der Gruppe, enthaltend -SO₂-CH=CH₂, -SO₂-CH₂CH₂-OSO₃H, -NH-CO-CBr=CH₂ und -NH-CO-CHBr-CH₂Br, enthält.

3. Verfahren gemäss einem der Ansprüche 1 und 2, **dadurch gekennzeichnet, dass** man die Lyocell-Cellulosefaser mit mindestens einer Verbindung der Formel (1) behandelt, worin B ein Rest der Formel 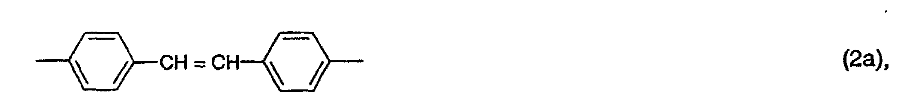 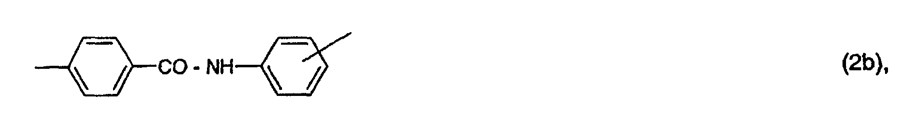 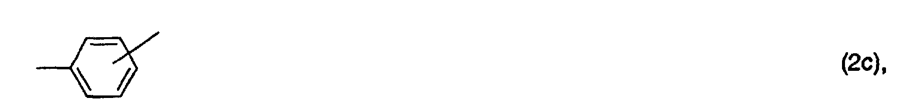 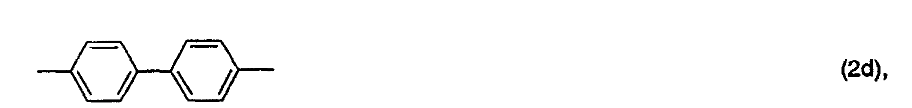 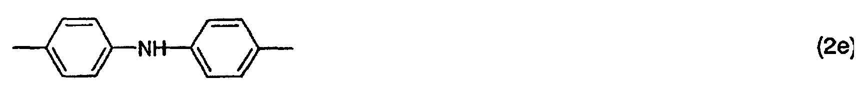 oder 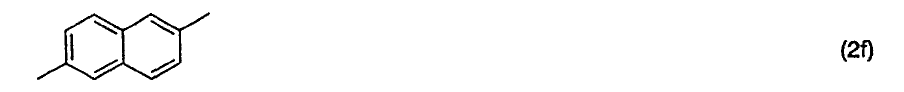 ist, wobei die Phenylreste in den Formeln (2a) bis (2f) ein- oder mehrfach durch Sulfo substituiert werden können.

4. Verfahren gemäss einem der Ansprüche 1 und 2, **dadurch gekennzeichnet, dass** man die Lyocell-Cellulosefaser mit mindestens einer Verbindung der Formel (1) behandelt, worin A₁, A₂, A₃ und A₄ -NH- bedeuten, B ein Rest der Formel 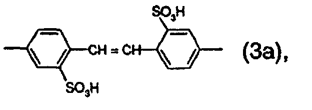 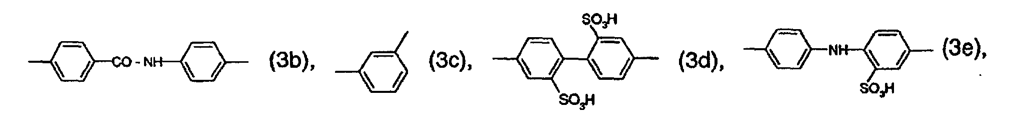 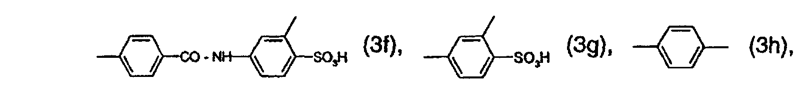
-CH₂-CH(CH₃)- (3i)
oder 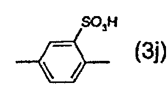 ist, R₁ Chlor, Fluor oder 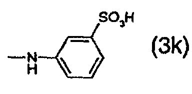 ist, R₂ Chlor oder Fluor ist, R₃ ein rest der Formel 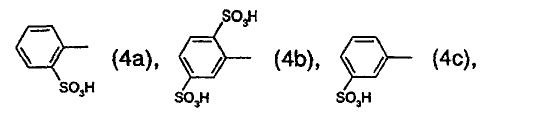 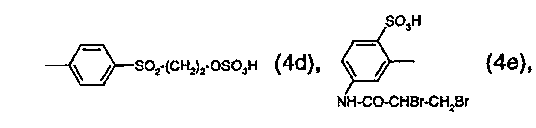 oder 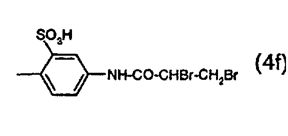 ist, und R₄ ein Rest der Formel (4a), (4b), (4d), (4e), (4f), , 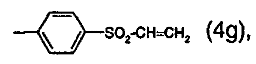 oder 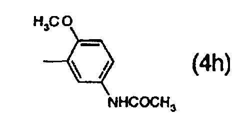 ist, oder A₃-R₃ und/oder A₄-R₄ Chlor bedeutet.

5. Verfahren gemäss einem der Ansprüche 1 und 2, **dadurch gekennzeichnet, dass** man zwischen 0,1 und 15 Gew.-% der Verbindung der Formel (1), bezogen auf das Gewicht der Faser, verwendet.

6. Verfahren gemäss einem der Ansprüche 1 und 2, **dadurch gekennzeichnet, dass** man die Verbindung der Formel (1) auf die frisch gesponnenen, noch nicht getrocknete Fasern, appliziert.

7. Verfahren gemäss einem der Ansprüche 1 und 2, **dadurch gekennzeichnet, dass** man die Verbindung der Formel (1) vor oder während eines Färbeprozesses auf die Fasern appliziert.

8. Verbindungen gemäss Anspruch 1 der Formeln 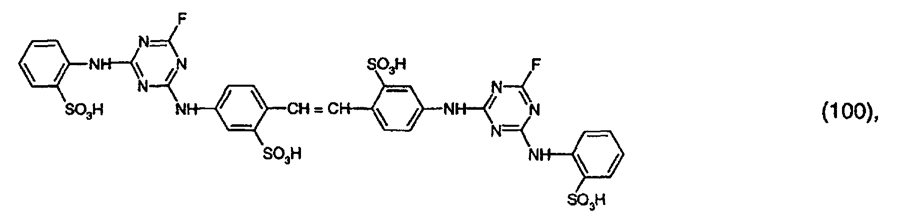 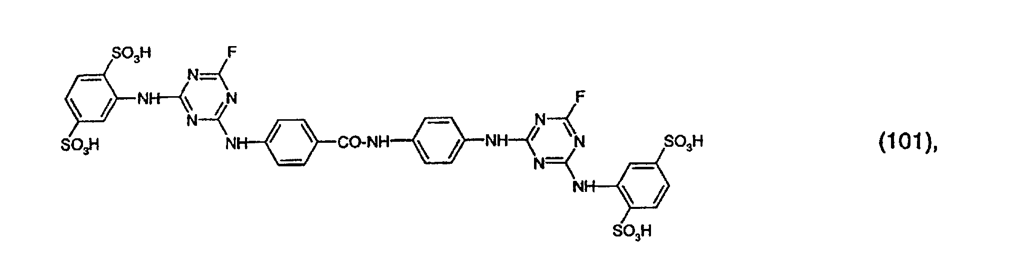 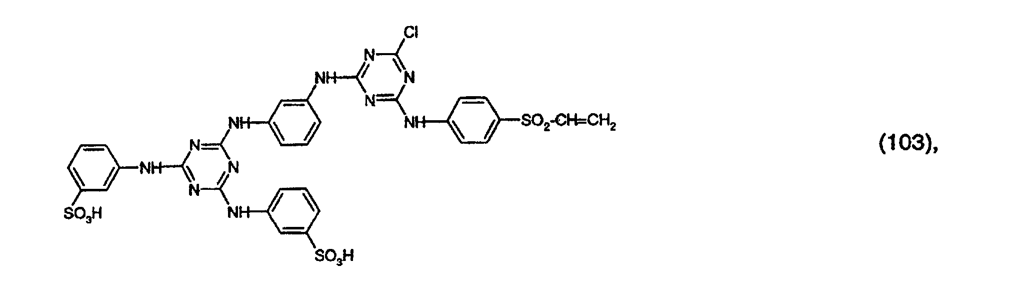 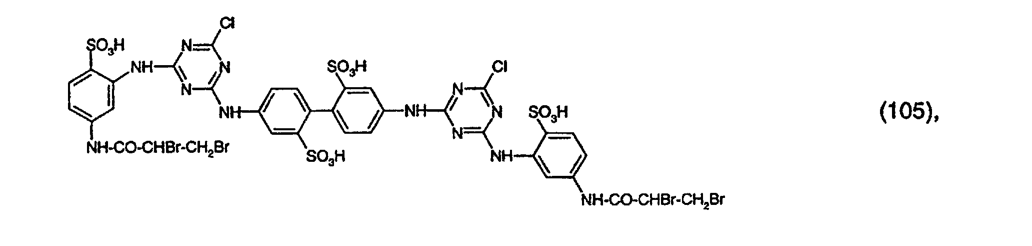 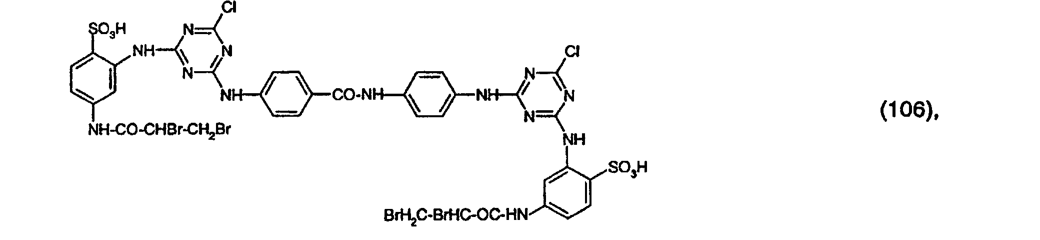 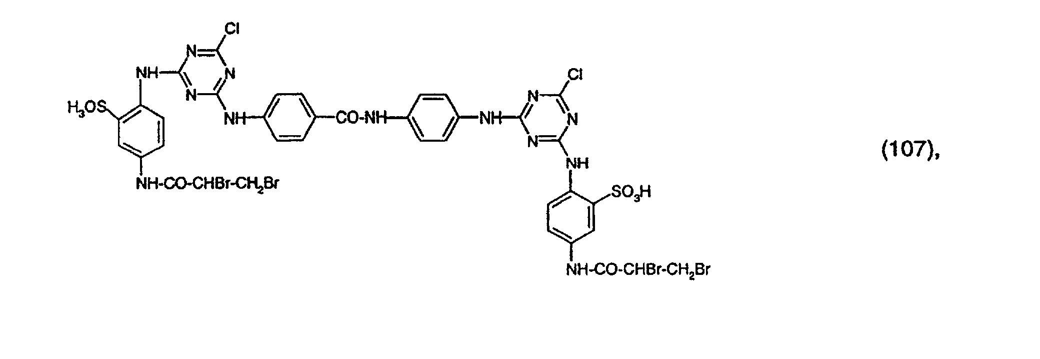 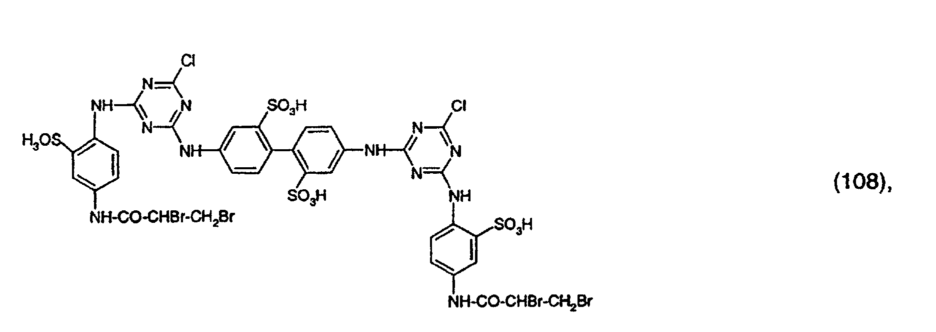 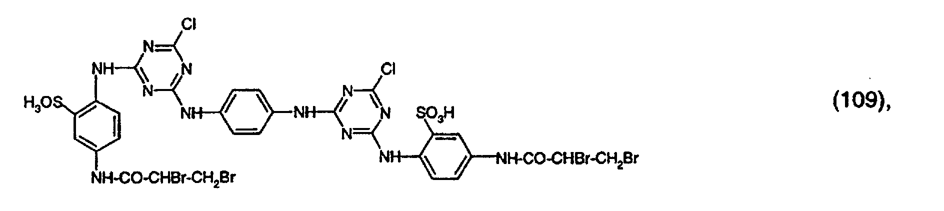 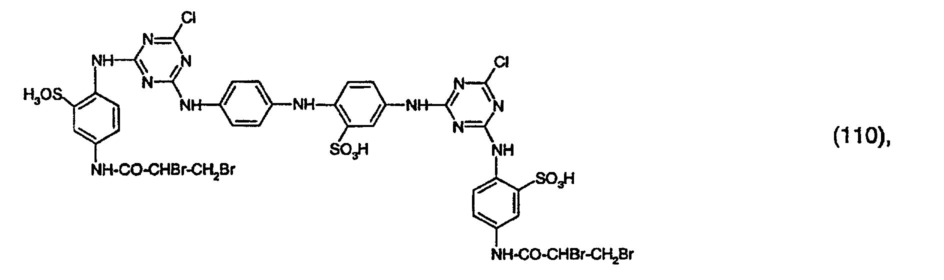 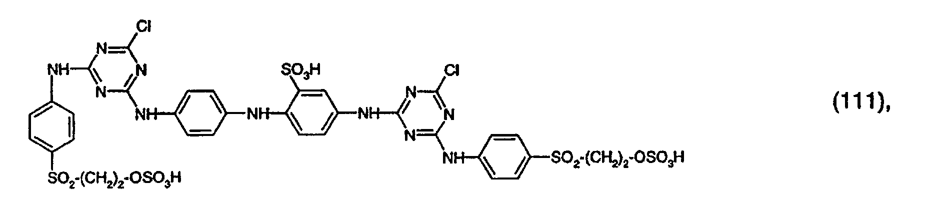 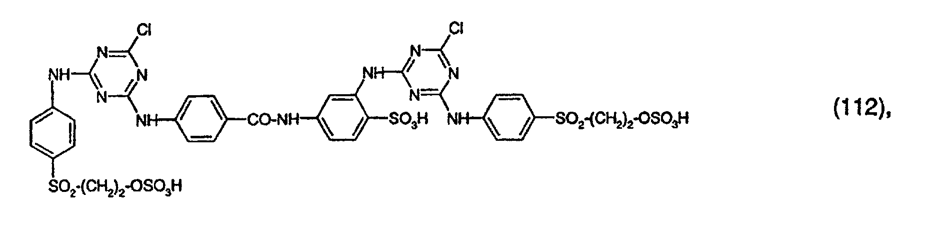 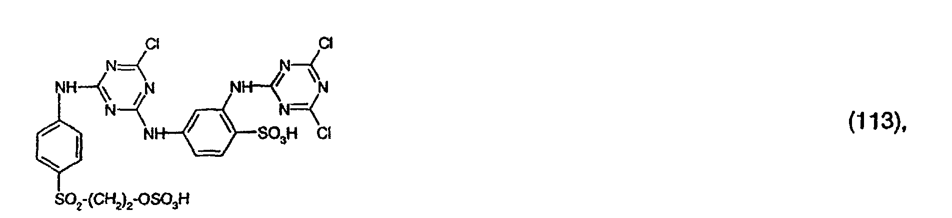 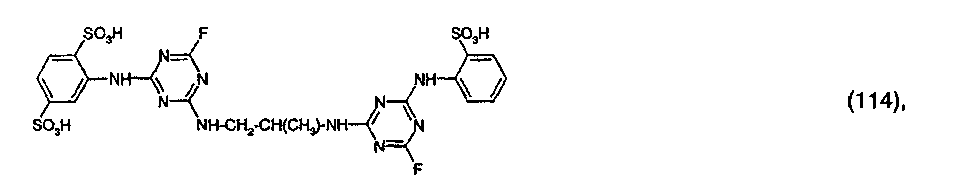 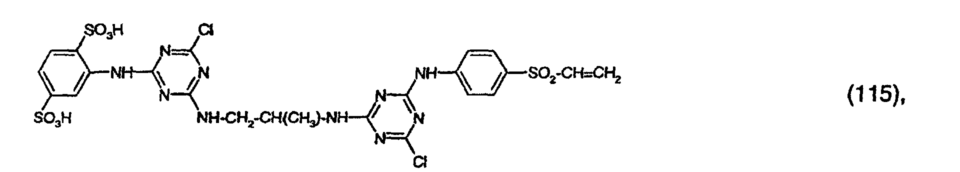 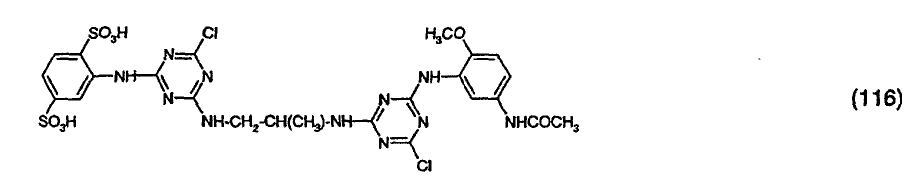 und

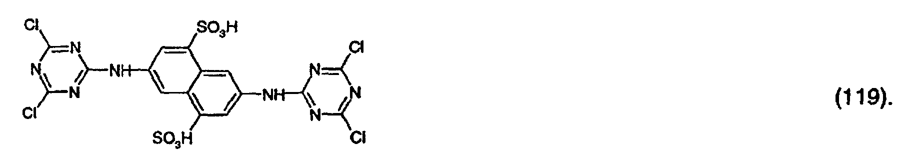

9. Verfahren zur Herstellung der Verbindungen der Formeln (100), (101), (103), (105) bis (116) und (119) gemäss Anspruch 8, **dadurch gekennzeichnet, dass** man ein Trichlortriazin der Formel 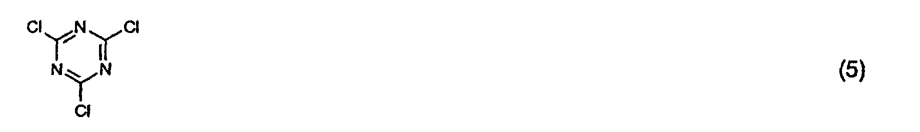 mit einer Verbindung der Formel 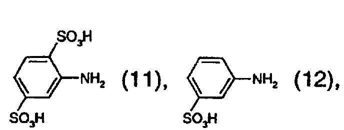 oder 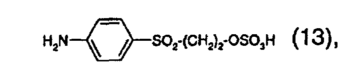 und anschliessend mit einer Verbindung der Formel 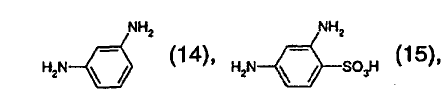 oder
H₂N-CH₂-CH(CH₃)-NH₂ (16),
umsetzt, und mit einem zweiten Trichlortriazin der Formel (5), welches gegebenenfalls vorgängig mit einer Verbindung der Formel 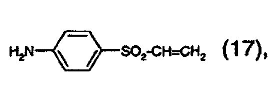 oder 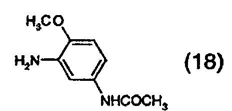 umgesetzt wurde, kondensiert, oder, falls A₃-R₃ = A₄-R₄, man Trichlortriazin der Formel (5) mit einer Verbindung der Formel (13), 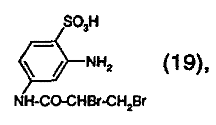 oder 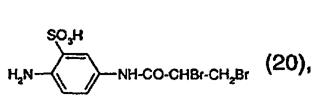 umsetzt und das entstehende Zwischenprodukt im Mol-Verhältnis 2:1 mit einer Verbindung der Formel 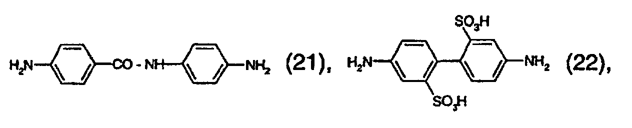 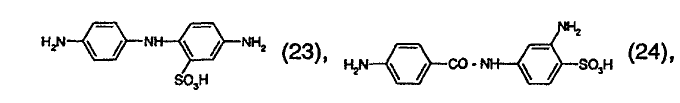 oder 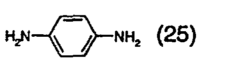 kondensiert, oder, falls A₃-R₃ = A₄-R₄ = Halogen, man Trichlortriazin der Formel (5) im Mol-Verhältnis 2:1 mit einer Verbindung der Formel (15) oder 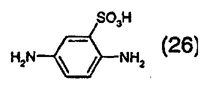 kondensiert, oder dass man ein Trifluorotriazin der Formel 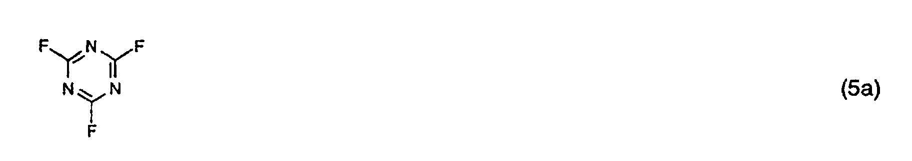 mit einer Verbindung der Formel 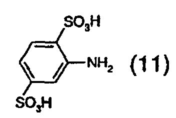 und anschliessend mit einer Verbindung der Formel (16), umsetzt, und mit einem zweiten Trifluorotriazin der Formel (5a), welches vorgängig mit einer Verbindung der Formel 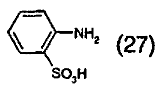 umgesetzt wurde, kondensiert, oder, falls A₃-R₃ = A₄-R₄, man Trifluorotriazin der Formel (5a) mit einer Verbindung der Formel (27) oder (11) umsetzt und das entstehende Zwischenprodukt im Mol-Verhältnis 2:1 mit einer Verbindung der Formel 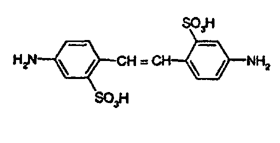 (28) oder (21), kondensiert.

10. Verwendung der Vebindungen gemäss Anspruch 1 zur Verringerung der Fibrillierung bei Lyocell-Cellulosefasern.

## Claims

1. A process for the treatment of Lyocell cellulose fibres, **characterized in that** the Lyocell cellulose fibre is treated with at least one compound of the formula 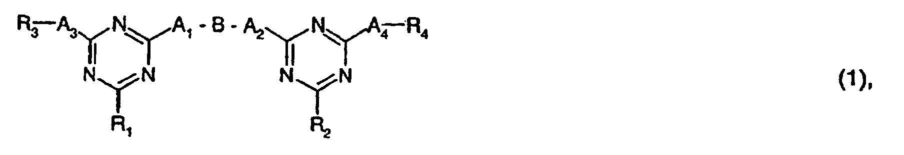 wherein
R₁ and R₂, independently of each other, each are a halogen or a phenylamino residue substituted by sulfo, wherein at least one of the two substituents R₁ and R₂ has the meaning of halogen,
R₃ and R₄, independently of each other, each are a phenyl that is unsubstituted or substituted by C₁-C₄-alkyl, C₁-C₄-alkoxy, acylamino, sulfo, -SO₂-CH=CH₂, -SO₂-CH₂CH₂-OSO₃H, -NH-CO-CBr=CH₂ or -NH-CO-CHBr-CH₂Br,
A₁ and A₂, independently of each other, each are -O-, -S- or -NR₅-, wherein R₅ stands for hydrogen or C₁-C₄-alkyl,
A₃ and A₄, independently of each other, each are -O-, -S- or -NR₅-, or -A₃-R₃ means halogen and/or -A₄-R₄ means halogen, wherein R₅ stands for hydrogen or C₁-C₄-alkyl and B is an aromatic binding link or -A₁-B-A₂- is a binding link of the formula -NH-CH₂-CH(CH₃)-NH-, with the proviso that the compound of formula (1) must contain at least one dihalogen triazine residue, at least two monohalogen triazine residues or at least one, preferably at least two, substituents from the group comprising -SO₂-CH=CH₂, -SO₂-CH₂CH₂-OSO₃H, -NH-CO-CBr=CH₂ and -NH-CO-CHBr-CH₂Br, which substituents are independent of each other.

2. A process according to claim 1, **characterized in that** the compound of formula (1) contains at least two substituents from the group comprising -SO₂-CH=CH₂, -SO₂-CH₂CH₂-OSO₃H, -NH-CO-CBr=CH₂ and -NH-CO-CHBr-CH₂Br, which substituents are independent of each other.

3. A process according to any of claims 1 and 2, **characterized in that** the Lyocell cellulose fibre is treated with at least one compound of formula (1), wherein B is a residue of the formula 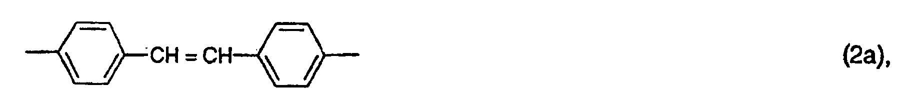 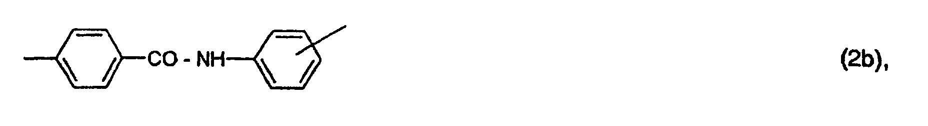 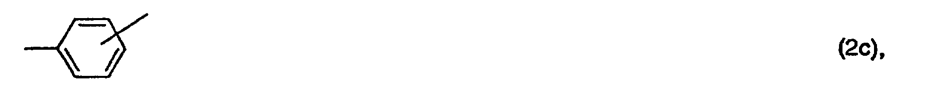 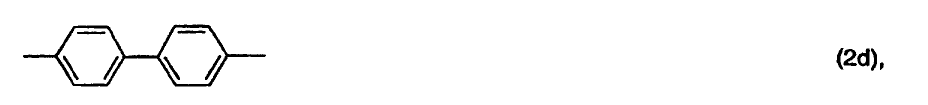 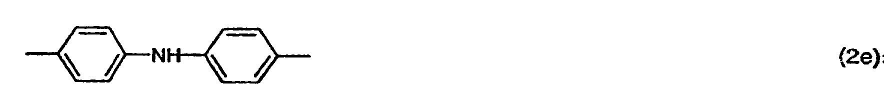 or 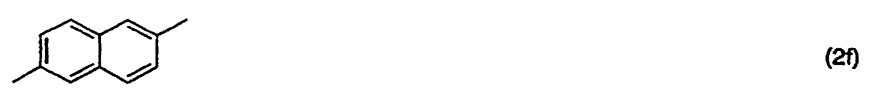 wherein the phenyl residues in formulae (2a) to (2f) may be substituted by sulfo one or several times.

4. A process according to any of claims 1 and 2, **characterized in that** the Lyocell cellulose fibre is treated with at least one compound of formula (1), wherein A₁, A₂, A₃ and A₄ mean -NH-, B is a residue of the formula 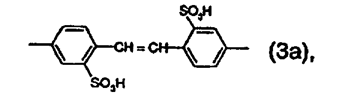 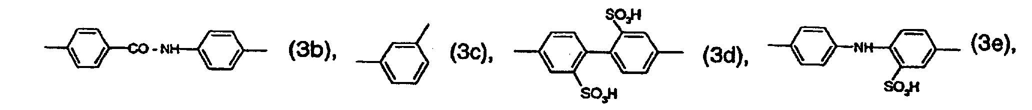 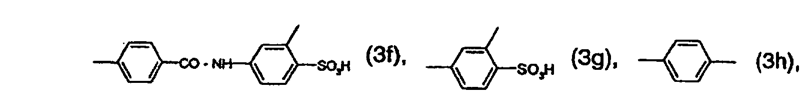
-CH₂-CH(CH₃)- (3i)
or 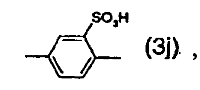 R₁ is chlorine, fluorine or 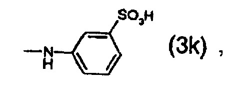 R₂ is chlorine or fluorine, R₃ is a residue of the formula 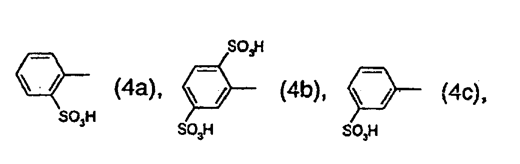 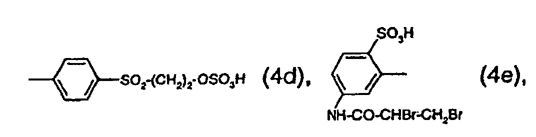 or 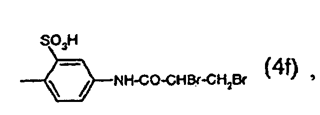 and R₄ is a residue of formula (4a), (4b), (4d), (4e), (4f) 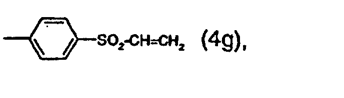 or 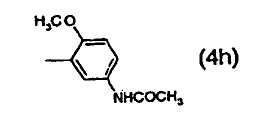 or A₃-R₃ and/or A₄-R₄ means chlorine.

5. A process according to any of claims 1 and 2, **characterized in that** between 0.1 and 15% by weight of the compound of formula (1), based on the weight of the fiber, is used.

6. A process according to any of claims 1 and 2, **characterized in that** the compound of formula (1) is applied onto the freshly spun, not yet dried fibres.

7. A process according to any of claims 1 and 2, **characterized in that** the compound of formula (1) is applied onto the fibres prior to or during the dyeing process.

8. Compounds according to claim 1 of the formulae 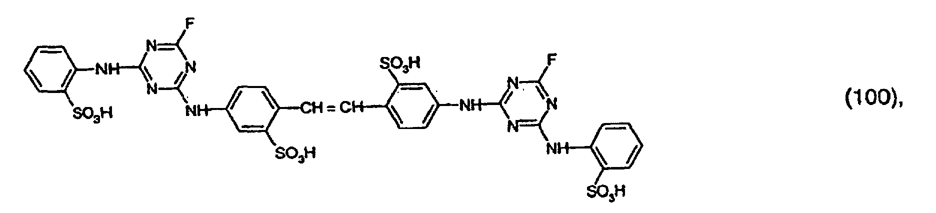 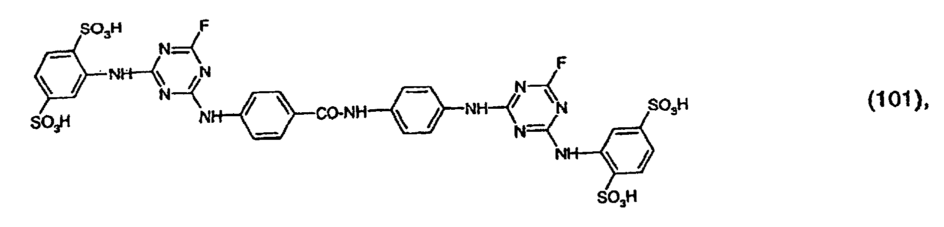 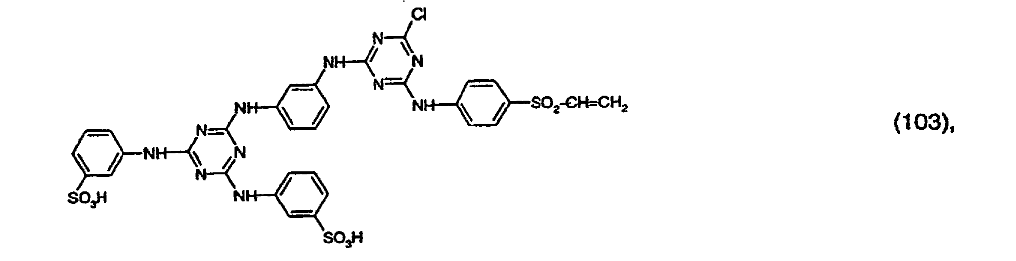 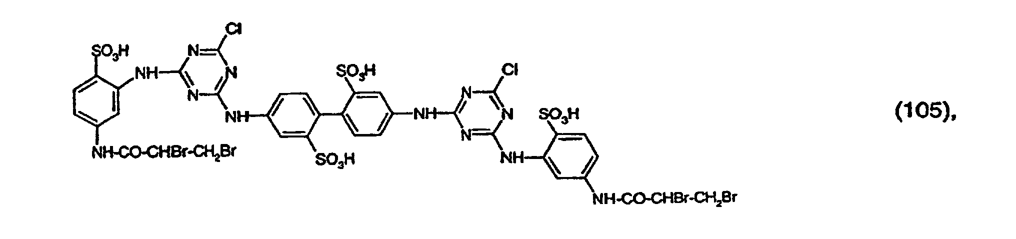 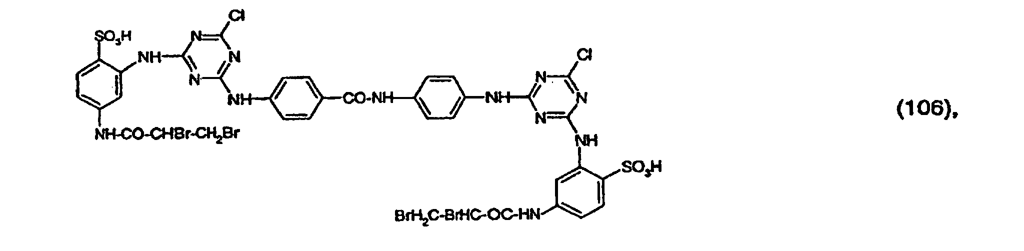 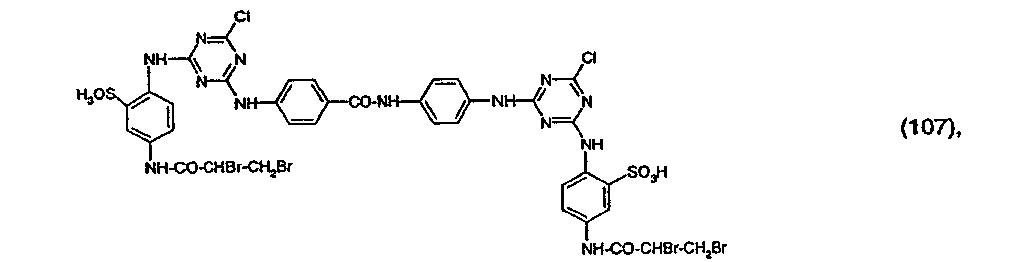 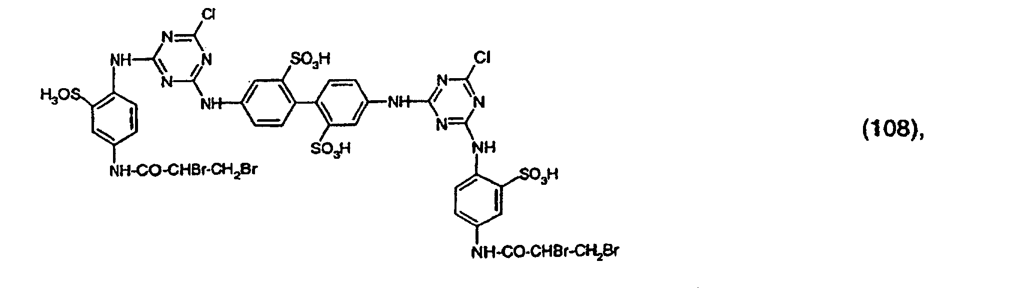 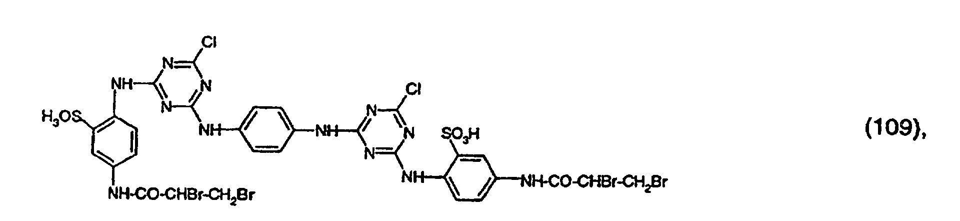 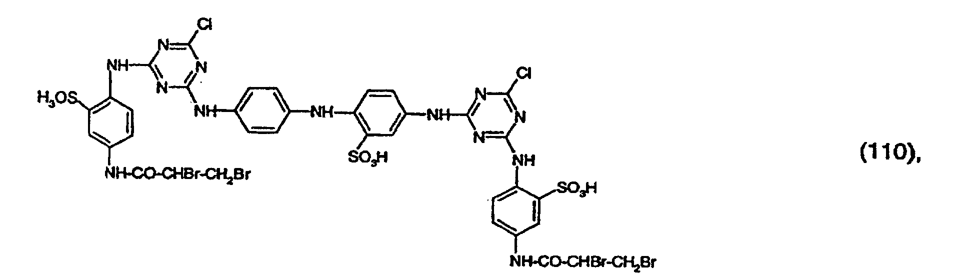 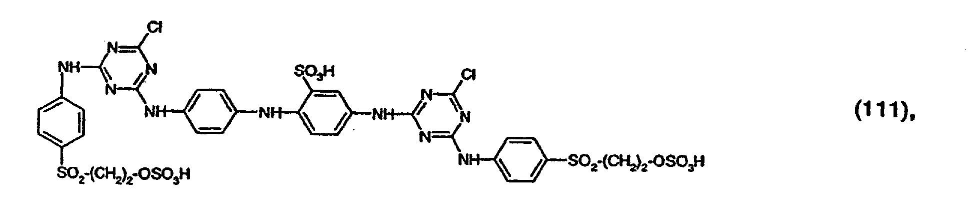 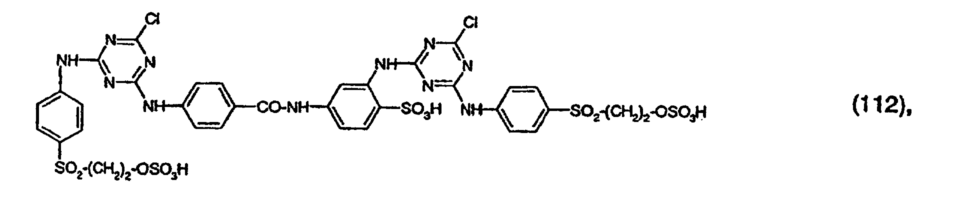 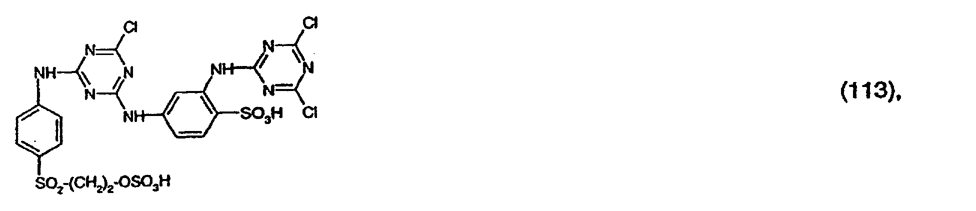 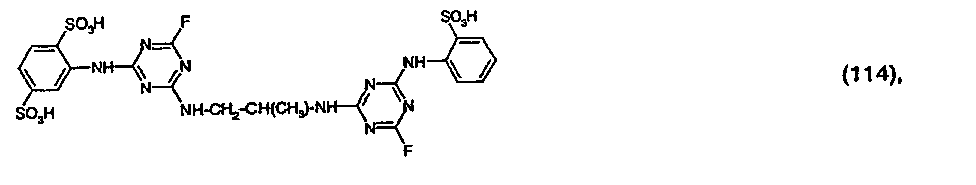 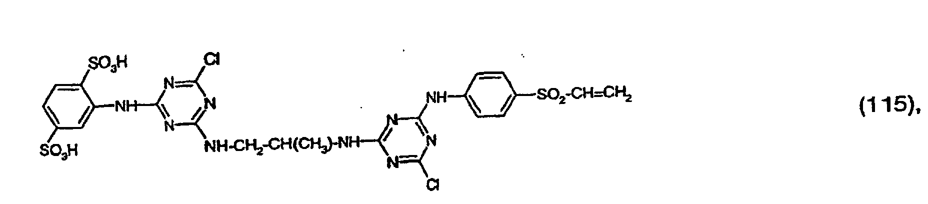 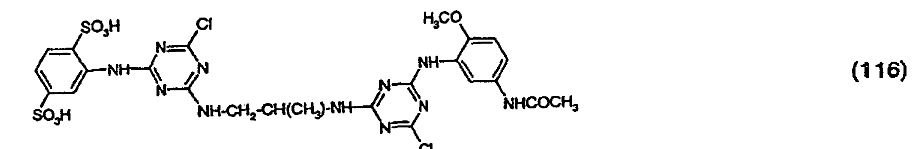 and

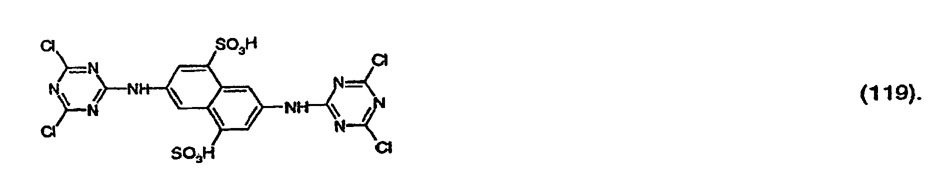

9. A process for preparing the compounds of formulae (100), (101), (103), (105) to (116) and (119) according to claim 8, **characterized in that** a trichlorotriazine of the formula 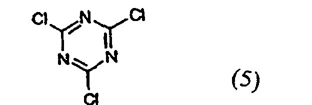 is reacted with a compound of the formula 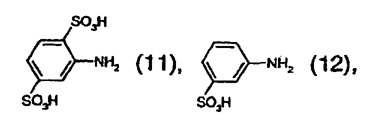 or 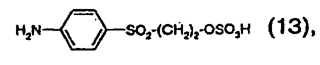 and subsequently with a compound of the formula 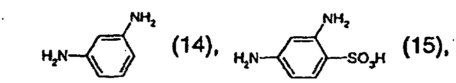 or
H₂N-CH₂-CH(CH₃)-NH₂ (16)
and is condensed with a second trichlorotriazine of formula (5) which, optionally, was previously reacted with a compound of the formula 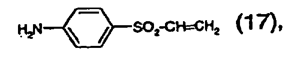 or 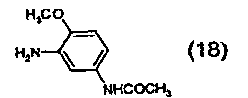 or, in case A₃-R₃=A₄-R₄, trichlorotriazine of formula (5) is reacted with a compound of formula (13), 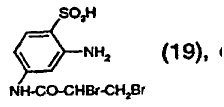 or 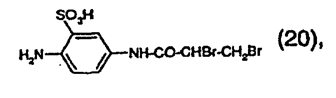 and the nascent intermediate is condensed at a molar ratio of 2:1 with a compound of the formula 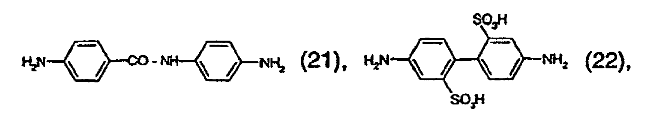 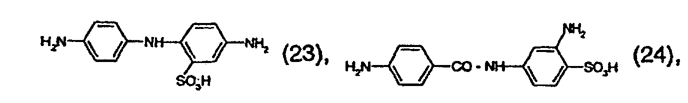 or 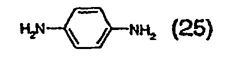 or, in case A₃-R₃=A₄-R₄=halogen, trichlorotriazine of formula (5) is condensed at a molar ratio of 2:1 with a compound of formula (15) or 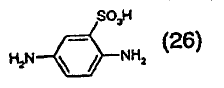 or that a trifluorotriazine of the formula 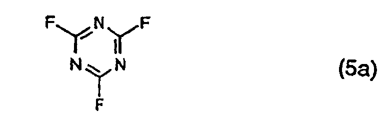 is reacted with a compound of the formula 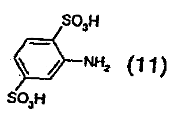 and subsequently with a compound of formula (16) and is condensed with a second trifluorotriazine of formula (5a) which was previously reacted with a compound of the formula 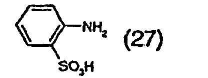 or, in case A₃-R₃=A₄-R₄, trifluorotriazine of formula (5a) is reacted with a compound of formula (27) or (11) and the nascent intermediate is condensed at a molar ratio of 2:1 with a compound of the formula 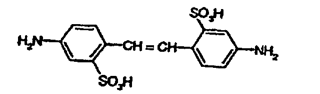 or (21).

10. The use of the compounds according to claim I for decreasing the fibrillation of Lyocell cellulose fibres.

## Revendications

1. Procédé de traitement de fibres de cellulose Lyocell, **caractérisé en ce que** les fibres de cellulose Lyocell sont traitées avec au moins un composé de la formule 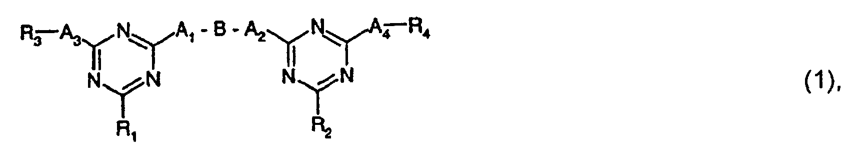 dans laquelle
R₁ et R₂ sont chacun indépendamment, un halogène ou un résidu phenyl-amino qui est sulfo-substitué, au moins un des deux substituants R₁ et R₂ étant un halogène,
R₃ et R₄ sont chacun indépendamment, un groupe phényle non substitué ou substitué par : alkyle en C₁ - C₄, alcoxy en C₁ - C₄, acylamino, sulfo, -SO₂-CH=CH₂, -SO₂-CH₂CH₂-OSO₃H, -NH-CO-CBr=CH₂ ou -NH-CO-CHBr-CH₂Br,
A₁ et A₂ sont chacun indépendamment -O-, -S- ou -NR₅-, où R₅ est l'hydrogène ou un alkyle en C₁ - C₄,
A₃ et A₄ sont chacun indépendamment -O-, -S- ou -NR₅-, ou -A₃-R₃ est un halogène et / ou A₄-R₄ est un halogène, où R₅ est l'hydrogène ou un alkyle en C₁ - C₄ et B est un groupe de liaison aromatique ou -A₁-B-A₂- est un groupe de liaison de la formule -NH-CH₂-CH(CH₃)-NH-, avec comme condition que le composé de la formule (1) doit comporter au moins un résidu dihalogénotriazine, au moins deux résidus monohalogénotriazine ou au moins un et, de préférence, au moins deux substituants choisis indépendamment dans le groupe comprenant -SO₂-CH=CH₂, -SO₂-CH₂CH₂-OSO₃H, -NH-CO-CBr=CH₂ et -NH-CO-CHBr-CH₂Br.

2. Procédé selon la revendication 1, **caractérisé en ce que** le composé de la formule (1) a au moins deux substituants choisis indépendamment dans le groupe comprenant -SO₂-CH=CH₂, -SO₂-CH₂CH₂-OSO₃H, -NH-CO-CBr=CH₂ et -NH-CO-CHBr-CH₂Br.

3. Procédé selon l'une des revendications 1 et 2, **caractérisé en ce que** les fibres de cellulose Lyocell sont traitées avec au moins un composé de la formule (1), dans laquelle B est un résidu de la formule : 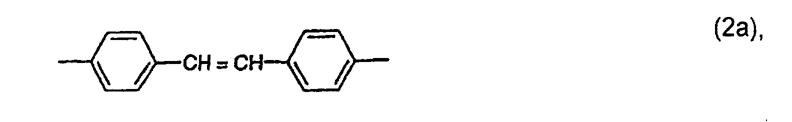 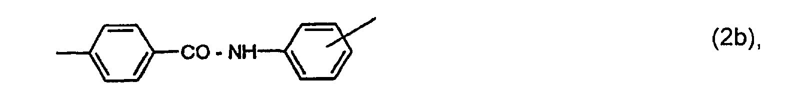 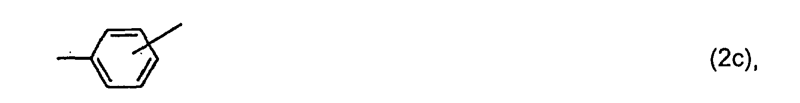 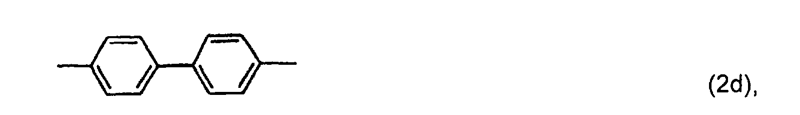 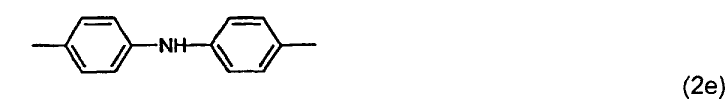 ou 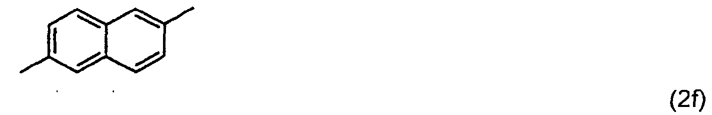 où le résidu phényle dans les formules (2a) à (2f) peut être substitué par un ou plusieurs groupes sulfo.

4. Procédé selon l'une des revendications 1 et 2, **caractérisé en ce que** les fibres de cellulose Lyocell sont traitées avec au moins un composé de la formule (1), dans laquelle A₁, A₂, A₃ et A₄ sont -NH-, B est un résidu de la formule 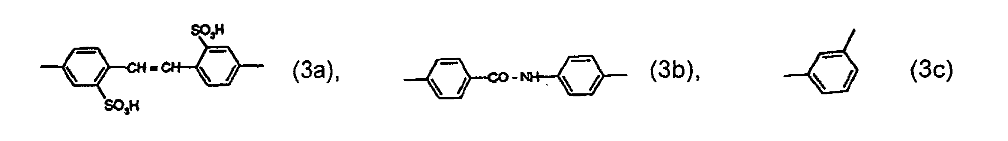 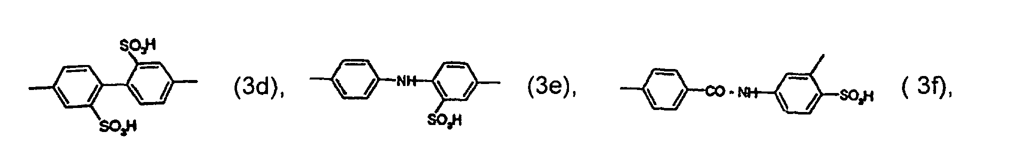 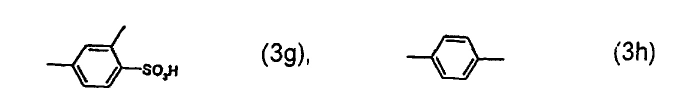
-CH₂-CH(CH₃)- (3i),
ou 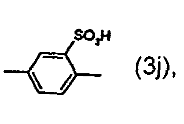 R₁ est le chlore, le fluor ou 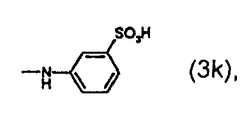 R₂ est le chlore ou le fluor, R₃ est un résidu de la formule 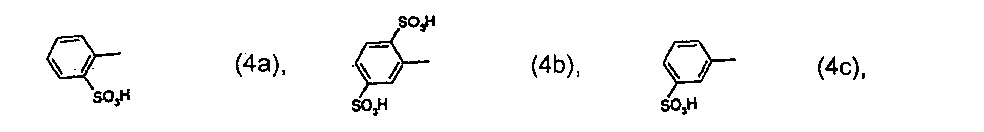 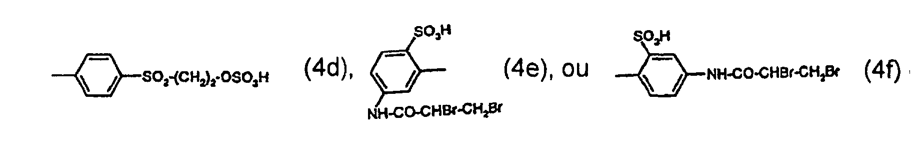 et R₄ est un résidu de la formule (4a), (4b), (4d), (4e), (4f), 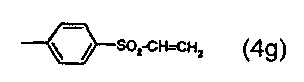 ou 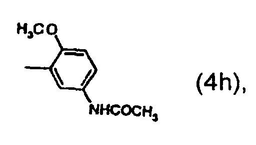 ou A₃-R₃ ou / et A₄-R₄ est / sont le chlore.

5. Procédé selon l'une des revendications 1 et 2, **caractérisé en ce que** le composé de la formule (1) est utilisé à raison de 0,1 à 15 % en poids des fibres.

6. Procédé selon l'une des revendications 1 et 2, **caractérisé en ce que** le composé de la formule (1) est appliqué sur des fibres fraîchement filées et non encore séchées.

7. Procédé selon l'une des revendications 1 et 2, **caractérisé en ce que** le composé de la formule (1) est appliqué sur les fibres avant ou pendant leur teinture.

8. Procédé selon la revendication 1 ayant la formule 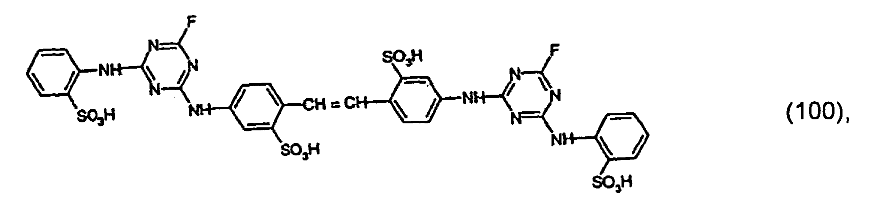 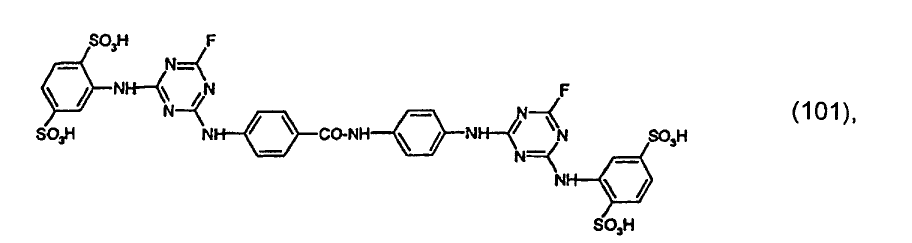 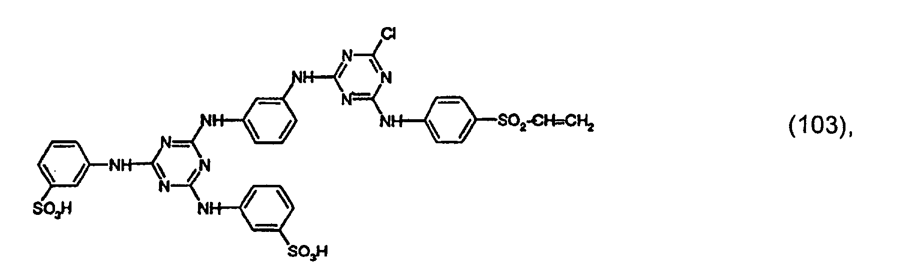 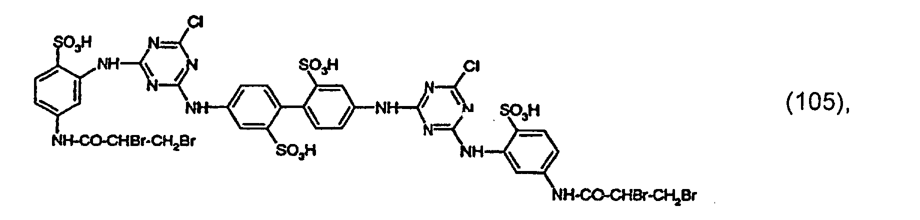 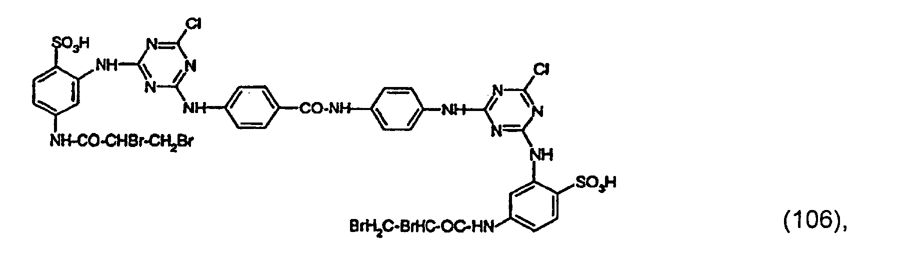 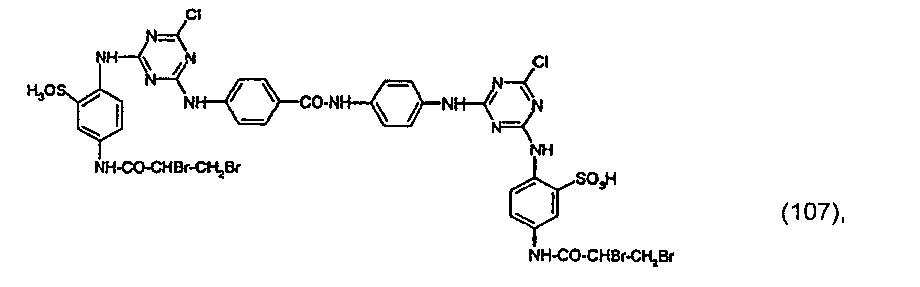 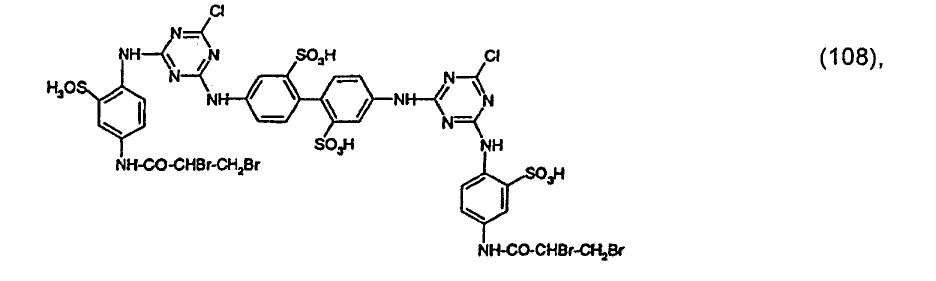 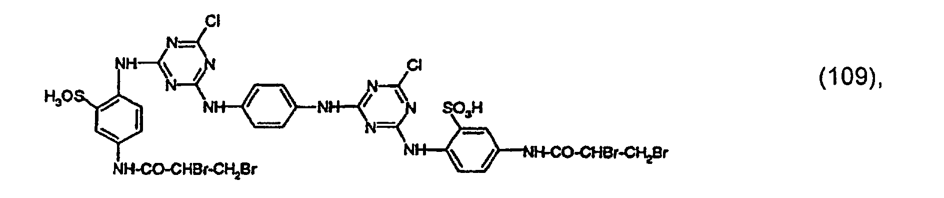 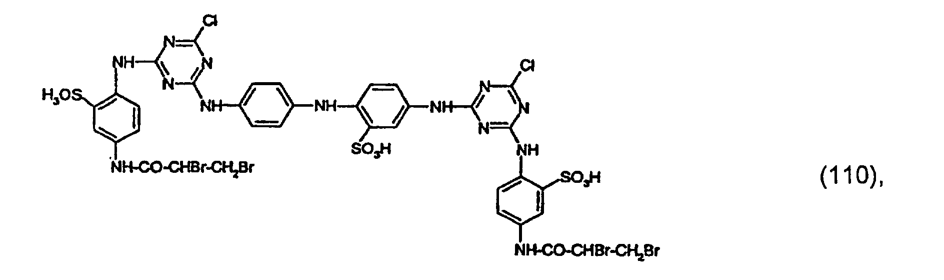 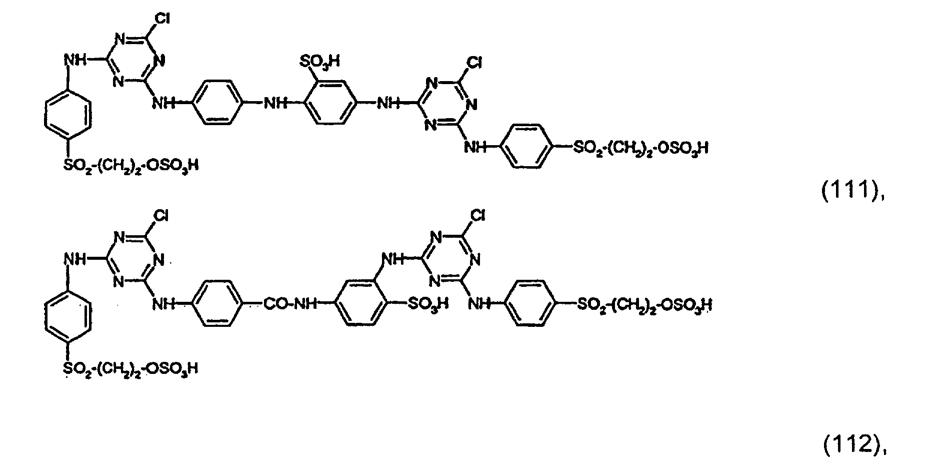 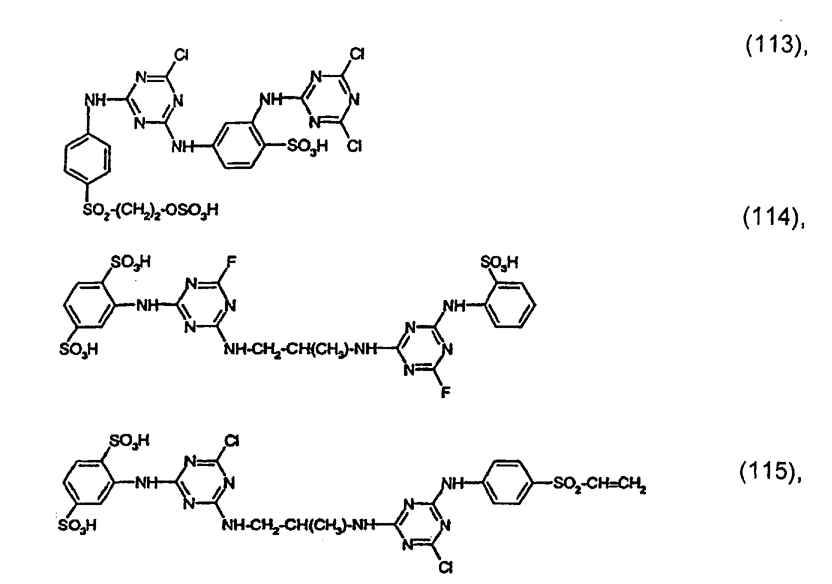 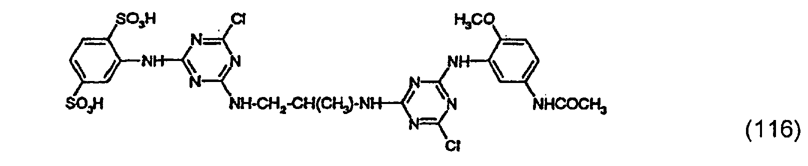 et

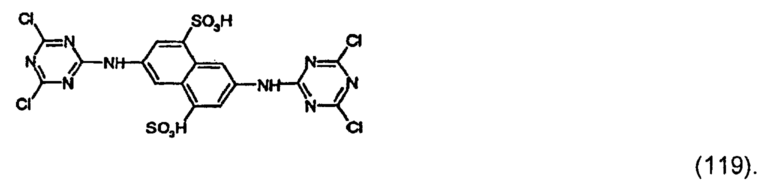

9. Procédé de préparation d'un composé de la formule (100), (101), (103), (105) à (116) et (119) de la revendication 8, **caractérisé en ce que** l'on fait réagir une trichlorotriazine de la formule 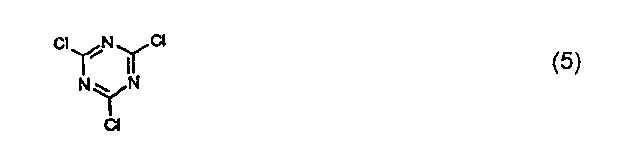 avec un composé de la formule 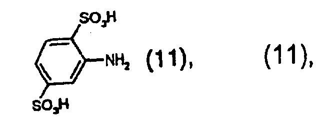 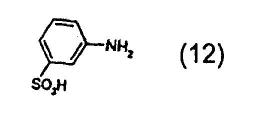 ou 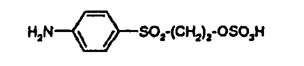 (13), puis avec un composé de la formule 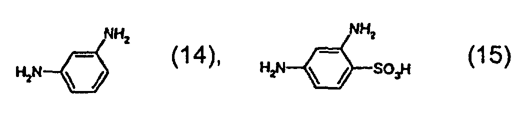 ou
H₂N-CH₂-CH(CH₃)-NH₂ (16)
et que le produit obtenu est condensé avec une seconde trichlorotriazine de la formule (5) que l'on avait fait réagir au préalable, le cas échéant, avec un composé de la formule 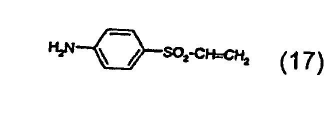 ou 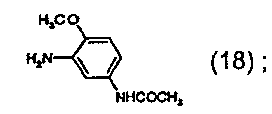 ou lorsque A₃-R₃ = A₄-R₄, que I' on fait réagir une trichlorotriazine de la formule (5) avec un composé de la formule (13), 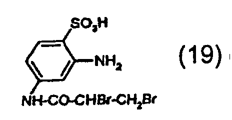 ou 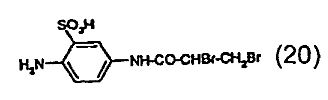 et que le produit intermédiaire obtenu est condensé dans un rapport molaire 2 : 1 avec un composé de la formule 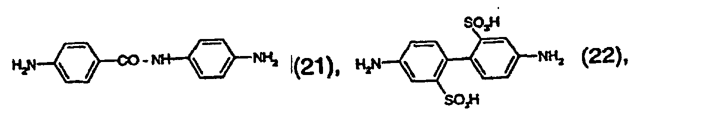 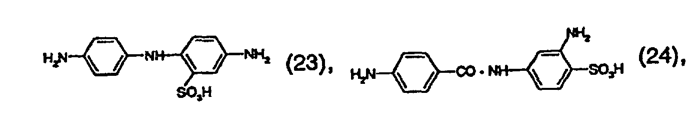 ou 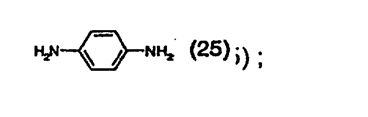 ou lorsque A₃-R₃ = A₄-R₄ = halogène, que l'on condense une trichlorotriazine de la formule (5) dans un rapport molaire 2 : 1 avec un composé de la formule (15) ou de la formule 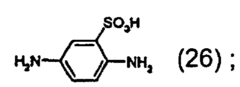 ou que l'on fait réagir une trifluorotriazine de la formule 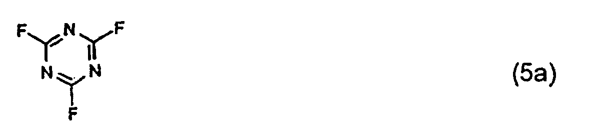 avec un composé de la formule 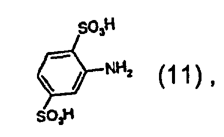 puis avec un composé de la formule (16) et que le produit obtenu est condensé avec une seconde trifluorotriazine de la formule (5a), que l'on avait fait réagir au préalable avec un composé de la formule 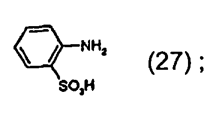 ou lorsque A₃-R₃ = A₄-R₄, que l'on fait réagir une trifluorotriazine de la formule (5a) avec un composé de la formule (27) ou (11) et que le produit intermédiaire obtenu est condensé dans un rapport molaire 2 : 1 avec un composé de la formule 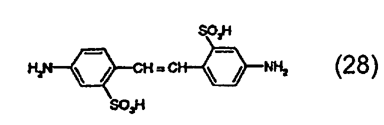 ou (21).

10. Utilisation d'un composé selon la revendication 1 pour diminuer la fibrillation de fibres de cellulose Lyocell.
